# EUROPEAN PATENT SPECIFICATION

(11) **EP 3 856 404 B1**
(45) Date of publication and mention of the grant of the patent: **24.07.2024**
(21) Application number: 19867427.7
(22) Date of filing: 27.09.2019
(51) Int. Cl.: C12Q 1/6806, G01N 33/543

(54) **TARGET CAPTURE IN MONODISPERSE DROPLETS**
ZIELERFASSUNG IN MONODISPERSEN TRÖPFCHEN
CAPTURE DE CIBLES DANS DES GOUTTELETTES MONODISPERSÉES

(30) Priority: 28.09.2018 US 201862738537 P
(43) Date of publication of application: 04.08.2021
(73) Proprietor: Fluent Biosciences Inc., Watertown, MA 02472 (US)
(72) Inventor: MELTZER, Robert, Watertown, Massachusetts 02472 (US); FONTANEZ, Kristina, Watertown, Massachusetts 02472 (US); SCHENK, Desiree, Watertown, Massachusetts 02472 (US); XUE, Yi, Watertown, Massachusetts 02472 (US)
(74) Representative: Graham Watt & Co LLP
(86) International application number: PCT/US2019/053426
(87) International publication number: WO 2020/069298

(56) References cited:
- WO-A1-2015/164212
- WO-A1-2018/022581
- WO-A1-2019/217552
- US-A1- 2018 105 808
- US-B2- 7 527 929
- ZILIONIS ET AL.: "Single- cell barcoding and sequencing using droplet microfluidics", NATURE PROTOCOLS, vol. 12, no. 1, 8 December 2016 (2016-12-08), pages 44 - 73, XP055367764, DOI: 10.1038/nprot.2016.154
- QI ET AL.: "Determination of the Bioavailability of Biotin Conjugated onto Shell Cross-Linked (SCK) Nanoparticles", J. AM. CHEM. SOC., vol. 126, no. 21, 7 May 2004 (2004-05-07), pages 6599 - 6607, XP055800195
- LOPES ET AL.: "Membrane nanoclusters of FcgammaRI segregate from inhibitory SIRPalpha upor activation of human macrophages", J CELL BIOL., vol. 216, no. 4, 13 March 2017 (2017-03-13), pages 1123 - 1141, XP055698708

## Description

### BACKGROUND OF THE INVENTION

Human body fluids contain a variety of diagnostic targets of medical importance. These include liquid biopsy targets such as circulating cells (tumor, fetal, or stem), cellular components (e.g. nuclei), cell-free nucleic acids, extracellular vesicles, and protein antigens which are being targeted for development of non-invasive diagnostics for a variety of cancers. These can also include biological targets indicative of disease such as prokaryotes, fungi, and viruses. However, the quantitative detection of proteins and nucleic acids at the single-cell level is challenging due to the tiny amount of protein available (Shani et al., (2017) "Abseq: Ultrahigh-throughput single cell protein profiling with droplet microfluidic barcoding." Scientific Reports volume 7, Article number: 44447). Efficient, target-specific, processing and barcoding of small quantities of nucleic acids is important for applications ranging from the assembly of uncultivable microbial genomes to the identification of cancer-associated mutations and remains a significant challenge. The present disclosure describes a flexible approach to capture and label targets of interest from biological samples, which leverages the particle-templated emulsification technique previously described (Hatori et al., (2018) "Particle-Templated Emulsification for Microfluidics-Free Digital Biology" Anal. Chem., 10.1021/acs.analchem.8b01759) and provides related advantages. In the prior art, WO 2015/164212 A1 relates to systems and methods for barcoding nucleic acids.

### SUMMARY OF THE INVENTION

The invention is defined in the appended claims. The methods and systems described herein provide capture template particles and an improved emulsion droplet-based target capture and barcoding method thereof. The capture template particles and methods disclosed herein allow capturing targets of interest from biological samples, and barcoding of specific nucleic acids contained in the captured targets. The nucleic acids can be contained within living or nonliving structures, including particles, viruses, and cells. The nucleic acids can include various biomolecules such as DNA or RNA.

In exemplary embodiments, the present disclosure provides but does not claim a capture template particle comprising a template particle comprising one or more tethering moieties, wherein each of the one or more tethering moieties is attachable to one or more target-specific elements.

In some embodiments, the one or more tethering moieties is selected from a capture moiety, and a capture element genetic identifier moiety. In some embodiments, the one or more target-specific elements are selected from a target-specific capture element, and a target-specific capture element genetic identifier. In some embodiments, the capture moiety comprises an Acrylate-terminated hydrocarbon linker with Biotin termination. In some embodiments, the capture moiety is attached to a target-specific capture element. In some embodiments, the target-specific capture element is selected from Poly-T polynucleotide sequences, Aptamers, and antibodies. In some embodiments, the capture element genetic identifier moiety comprises an Acrylate terminated hydrocarbon linker with a terminal adapter polynucleotide. In some embodiments, the capture element genetic identifier moiety is attached to a target-specific capture element genetic identifier. In some embodiments, the target-specific capture element genetic identifier is an oligonucleotide and comprises elements selected from a universal adaptor sequence, template-type element, template-ID element, target-specific capture element, and combinations thereof. In some embodiments, the template particle further comprises a hydrogel selected from agarose, alginate, a polyethylene glycol (PEG), a polyacrylamide (PAA), Acrylate, Acrylamide/bisacrylamide copolymer matrix, and combinations thereof. In some embodiments, the template particle is sized to the desired diameter by microfluidic co-flow with immiscible oil. In other embodiments, the template particle is sized to a desired diameter using any method known in the art and/or discussed herein.

The present disclosure provides but does not claim a method of target capture and barcoding in monodisperse droplets comprising:
1. combining a plurality of capture template particles according to a method described herein with a first fluid to provide a first mixture, wherein the first fluid comprises a plurality of target particles;
2. incubating the first mixture to allow association of the plurality of target particles to the plurality of capture template particles, thereby a portion of the plurality of target particles becomes associated to the capture template particles;
3. centrifuging the first mixture to generate supernatant and pellet;
4. removing the supernatant to separate the plurality of capture template particles and associated target particles from the solution of the first mixture;
5. washing and resuspending the pellet in reaction buffer to provide a second mixture;
6. combining the second mixture with a second fluid to provide a third mixture, wherein the second fluid is immiscible with the second mixture; and shearing the third mixture such that a plurality of the capture template particles are encapsulated in a plurality of monodisperse droplets in the second fluid, thereby providing a plurality of monodisperse droplets comprising second fluid, one of the capture template particles, and one of the plurality of target particles associated to said capture template particles.

In some embodiments, the second fluid comprises an oil. In some embodiments, the oil comprises a fluorocarbon oil, a hydrocarbon oil, silicone oil, or a combination thereof. In some embodiments, the first fluid comprises a biological sample. In some embodiments, the biological sample is a body fluid. In some embodiments, the body fluid comprises target particles selected from circulating cells, cellular components, cell-free nucleic acids, extracellular vesicles, protein antigens, prokaryotes cells, fungi, viruses, and combinations thereof. In some embodiments, the body fluid is a human body fluid. In some embodiments, the association of the plurality of target particles to the plurality of capture template particles is target-specific and is generated via the target-specific capture element. In some embodiments, the reaction buffer is a PCR reaction buffer comprising PCR reagents. In some embodiments, the reaction buffer is a reverse transcription buffer comprising reverse transcription reagents. In some embodiments the reaction buffer further comprises lysis buffer. In some embodiments, the associated target particles encapsulated in the plurality of monodisperse droplets are lysed wherein the lysis does not disturb the encapsulation of the plurality of the monodisperse droplets. In some embodiments, the lysis releases nucleic acids present in the associated target particles. In some embodiments, a portion of the released nucleic acids associate to the capture template particles via the capture element genetic identifier in a target-specific manner. In some embodiments, the lysis reagent, (unlimiting examples of a lysis reagent include Triton X-100, SDS, Sarkosyl, perfluoropolyether (PFPE), carboxylic acid, ammonium carboxylate, or potassium carboxylate) is present in the second fluid and triggers cell lysis upon emulsification (shearing of the third mixture as described above in step 6 of the method of target capture and barcoding in monodisperse droplets).

In some embodiments, the nucleic acids associated to the plurality of capture template particles comprise mRNA molecules. In some embodiments, the mRNA molecules associated to the plurality of capture template particles are reverse transcribed to create a complementary cDNA sequence. In some embodiments, the encapsulation of the plurality of capture template particles is broken with a PCR compatible chemistry. In some embodiments, a PCR is performed with a universal adaptor-specific sequence to release nucleic acids associated to the plurality of capture template particles from said plurality of capture template particles. In some embodiments, the PCR products may be used to prepare a sequencing library.

The present disclosure provides but does not claim a method of preparation of capture template particles comprising:
1. preparation of template particles by combining Acrylamide/bisacrylamide copolymer matrix, Acrylate-terminated hydrocarbon linker with Biotin termination, and Acrylate terminated hydrocarbon linker with terminal adapter polynucleotide;
2. sizing the template particles to the desired diameter by microfluidic co-flow into an immiscible oil phase;
3. combining the template particles with target-specific capture elements, thus causing the attachment of the target-specific capture elements to the template particles;
4. combining the template particles with target-specific capture element genetic identifiers, thus causing the attachment of the capture element genetic identifiers to the template particles; and, thus generating the capture template particles.

In some embodiments, the target-specific capture elements comprise streptavidin, and the target-specific capture elements attach to the template particles via biotin-streptavidin affinity. In some embodiments, the target-specific capture element genetic identifiers comprise universal adaptor oligonucleotide sequence, and said target-specific capture element genetic identifiers attach to the template particles by complementary strand interaction between the universal adaptor oligonucleotide sequences.

In another aspect, the present disclosure provides but does not claim a method for generating a plurality of partitions, comprising: (a) providing a container containing a composition comprising (i) a first liquid, (ii) a second liquid, (iii) one or more target particles comprising a polynucleotide, and (iv) a plurality of capture template particles, wherein each target particle of the one or more target particles comprising a polynucleotide is attached to an outer surface of a capture template particle of the plurality of capture template particles; wherein the second liquid is immiscible with the first liquid; and (b) agitating the container or the composition in its entirety to generate a plurality of partitions within the second liquid, wherein at least one partition of the plurality of partitions comprises (i) at least a portion of the first liquid, (ii) a single capture template particle of the plurality of capture template particles, and (iii) a single target particle comprising a polynucleotide of the one or more target particles comprising a polynucleotide; and wherein the single target particle comprising a polynucleotide is in contact with an inner surface of the partition.

In some embodiments, the single target particle is a single cell or exosome. In some embodiments, the method further comprises lysing the single cell or exosome. In some embodiments, each capture template particle of the plurality of capture template particles further comprises a capture element genetic identifier moiety. In some embodiments, the capture element genetic identifier moiety comprises a target-specific sequence. In some embodiments, the capture element genetic identifier moiety further comprises a barcode sequence. In some embodiments, the capture element genetic identifier moiety further comprises an adaptor sequence. In some embodiments, the second liquid comprises an oil.

### BRIEF DESCRIPTION OF THE DRAWINGS

The novel features of the invention are set forth with particularity in the appended claims. A better understanding of the features and advantages of the present invention will be obtained by reference to the following detailed description that sets forth illustrative embodiments, in which the principles of the invention are utilized, and the accompanying drawings of which:

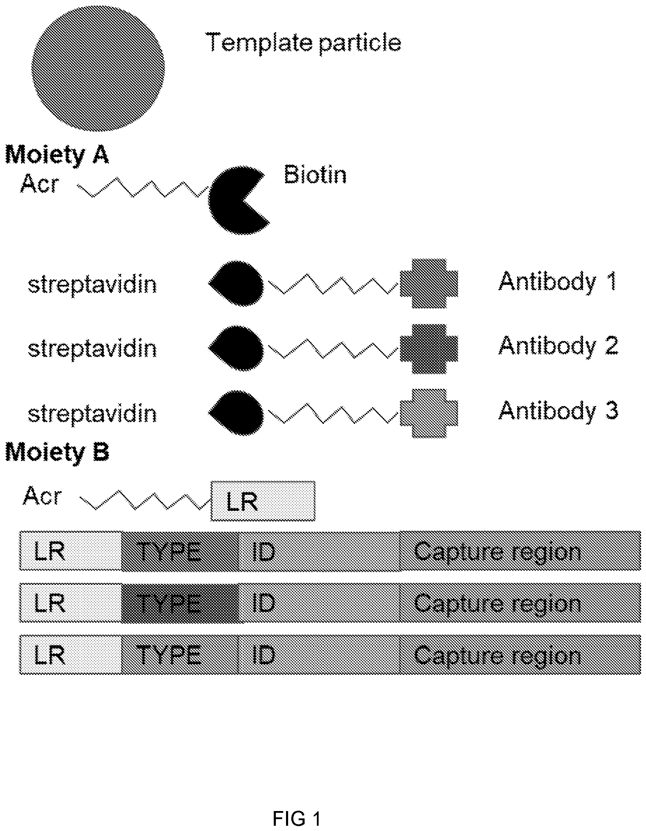

**FIG. 1** is a schematic depicting the elements of a capture template particle according to an embodiment of the present disclosure. A schematic representation of a capture template particle is shown. The capture template particle may comprise a tethering moiety, or tethering moieties, including a capture moiety (referred to as "Moiety A"), and capture element genetic identifier moiety (referred to as "Moiety B"). In the embodiment shown, the capture moiety comprises Acrylate-terminated hydrocarbon linker with Biotin termination, and may attach target-specific capture elements, such as a streptavidin-conjugated antibody. Target-specific, streptavidin-conjugated antibodies are illustrated: Antibody 1, 2, and 3. The capture element genetic identifier moiety comprises a hydrocarbon linker with a terminal adapter polynucleotide, wherein the terminal adapter polynucleotide comprises a linker sequence and universal adaptor oligonucleotide (the linker sequence and universal adaptor oligonucleotide are referred to herein as "LR"), and may attach target-specific capture element genetic identifier comprising an LR oligonucleotide via complementary strand interaction. The capture element genetic identifier depicted is an oligonucleotide comprising an LR, a template-type element (referred to as "TYPE"), a template ID element (referred to as "ID"), and target-specific capture element (referred to as "Capture region").

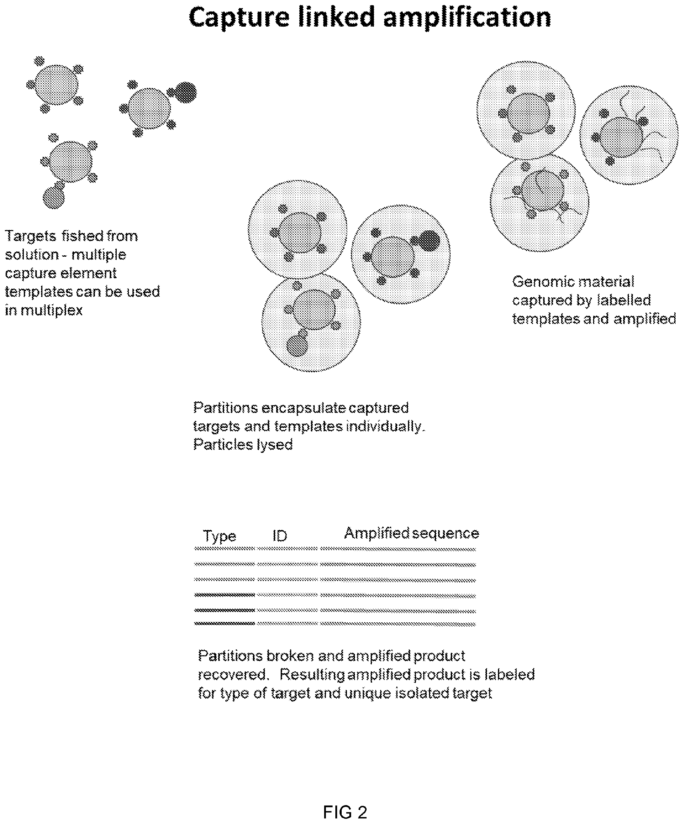

**FIG. 2** is a schematic generally showing the method of target capture and barcoding according to an embodiment of the present disclosure. Depicted are capture template particles comprising target-specific antibodies (represented schematically by gray circles surrounded by crosses, left panel of FIG. 2). The capture template particles are combined with target particles (depicted as small circles, left panel of FIG. 2) allowing for target-specific capture of a portion of the target particles. Each capture template particle, whether or not associated with a captured target particle, is encapsulated in monodisperse droplets (depicted by the larger gray circles, middle panel of FIG. 2). Associated target particles are then lysed within the encapsulation to release nucleic acids (depicted by black lines within the monodisperse droplet, right panel of FIG. 2). A portion of the nucleic acids then associate in a site-specific manner with the capture template particle's target-specific capture element, and associated nucleic acids are amplified. The amplification products are labeled for the target-specific capture element used (depicted by "Type") and for the specific target particle they originated from ("ID"). Amplified sequence is shown as the segment attached to the "ID" segment in the figure.

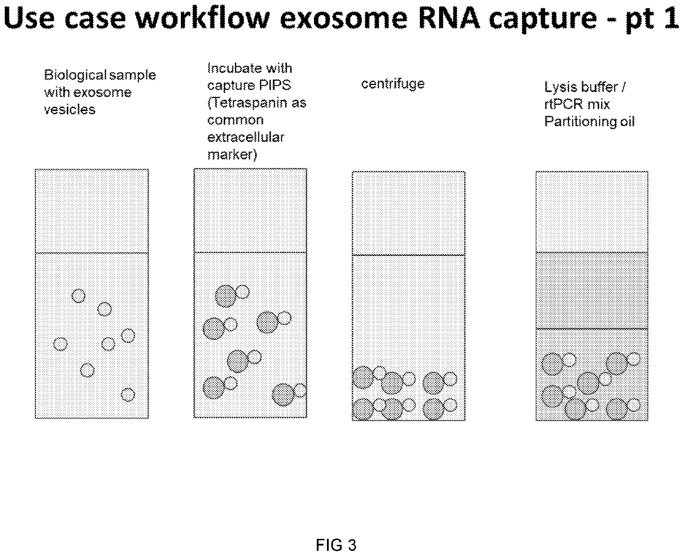

**FIG. 3** is a part 1 of a schematic showing a case workflow of the method of target capture and barcoding in monodisperse droplets according to an embodiment of the present disclosure. A biological sample comprising exosome vesicles (gray circles) is incubated with capture template particles (referred to as "capture PIPS") to provide a first mixture. In the embodiment depicted, the capture template particles comprise anti-Tetraspanin antibodies which allow for target-specific association of the exosomes with the capture template particles. The capture template particles further comprise a capture element genetic identifier comprising a Poly-T sequence. Each target-specific association comprises one exosome and one capture template particle. The mixture is centrifuged and associated exosomes and target capture particles are pelleted. The supernatant is discarded (not shown) and the pellet is resuspended in a reaction buffer comprising lysis buffer and rtPCR reagent mix to provide a second mixture. Oil ("Partitioning oil") is added to the second mixture to provide a third mixture.

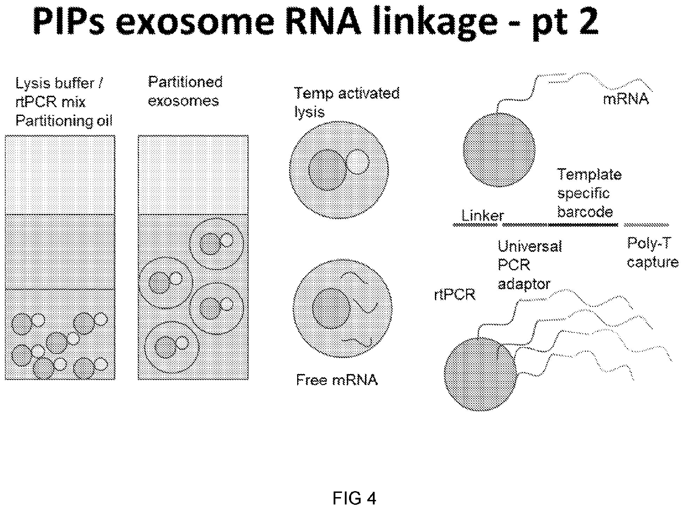

**FIG. 4** is a part 2 of a schematic showing a case workflow of the method of target capture and barcoding in monodisperse droplets according to an embodiment of the present disclosure. Shearing or partitioning the third mixture such that the capture template particles and associated exosomes are encapsulated in a plurality of monodisperse droplets in the partitioning oil. Each capture template particle and associated exosome are encapsulated in a single monodisperse droplet. Associated exosomes are then lysed, using temperature activated lysis, to release their contents, including nucleic acids (depicted as black lines), without breaking the monodisperse droplets encapsulation. Released mRNA molecules can then may associate with the Poly-T sequences ("Poly-T capture") of the capture template particle's capture element genetic identifier. The other elements of the depicted capture element genetic identifier include an LR oligonucleotide comprising a linker sequence (referred to as "Linker", depicted in dark gray) and universal adaptor oligonucleotide (referred to as "universal PCR adaptor"), and a template ID element (referred to as "Template specific barcode"). A reverse transcription reaction is then performed to generate cDNA templates of the associated mRNAs. FIG. 4 depicts multiple cDNA molecules attached to the capture template particle's multiple capture element genetic identifiers.

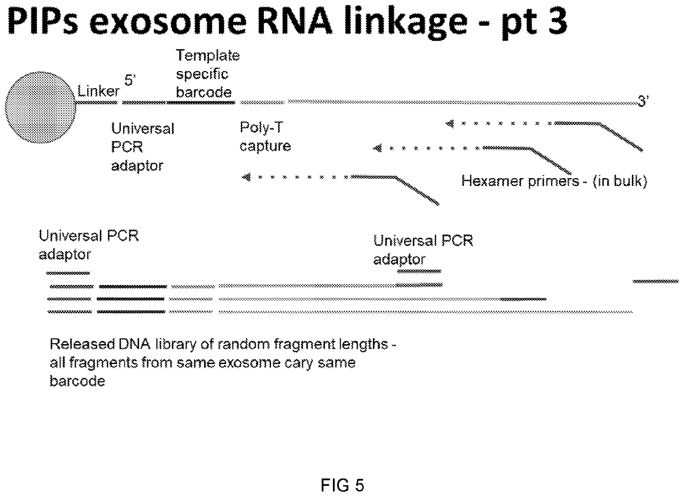

**FIG. 5** is a part 3 of a schematic showing a case workflow of the method of target capture and barcoding in monodisperse droplets according to an embodiment of the present disclosure. An amplification reaction of the cDNAs of FIG. 4 is illustrated (broken lines with arrowheads), using hexamer primers (referred to as "Hexamer primers - (in bulk)") comprising a universal adaptor sequence "tail" (referred to as "Universal PCR adaptor", depicted in red) to create amplification products of variable target sequence length. The amplification products of the hexamer primers contain the hexamer's tail which comprises the universal adaptor element on their 3' ends, and the ID, TYPE, and Universal elements, derived from the capture element genetic identifier, on their 5' ends. All the fragments (DNA amplification products) from same exosome carry the same template ID element (also referred to as "barcode").

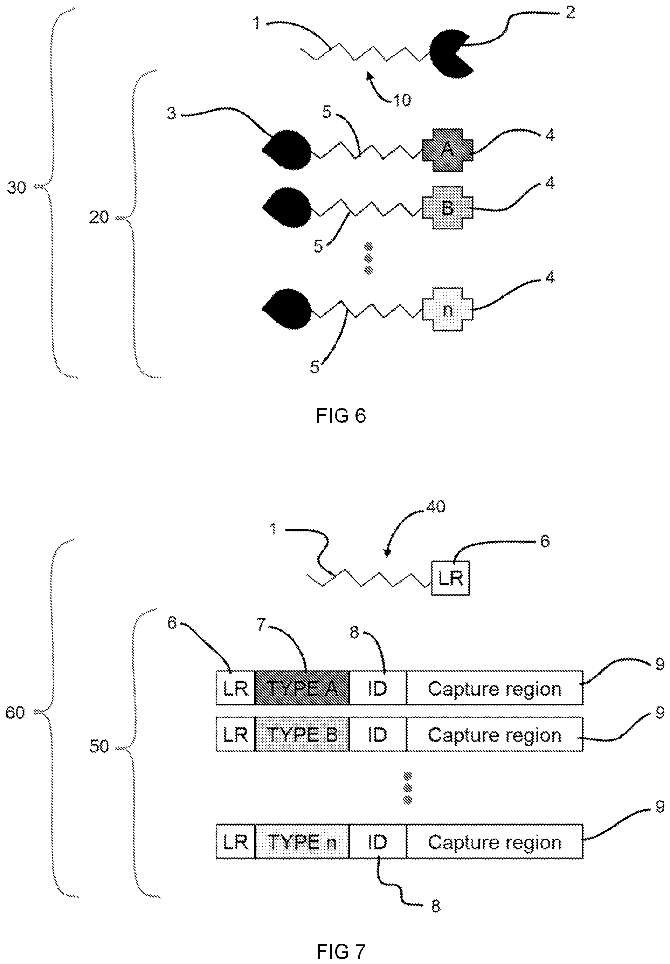

**FIG. 6** is a schematic depicting an embodiment of the capture moiety of the capture template particle. In the embodiment shown, the capture moiety comprises Acrylate-terminated hydrocarbon linker with Biotin termination, and is attachable to target-specific capture elements, such as a streptavidin-conjugated antibody. In the embodiment illustrated the target-specific, streptavidin-conjugated antibodies represented by the letters "A" and "B". A and B represent any chosen and suitable antibody and practically infinite versions of target-specific capture elements can be created using various antibodies as indicated by ellipsis and illustrated by antibody "n".
**FIG. 7** is a schematic depicting the capture element genetic identifier moiety of the capture template particle. The capture element genetic identifier moiety comprises an Acrylate hydrocarbon linker with a terminal adapter polynucleotide, wherein the terminal adapter polynucleotide comprises a linker sequence and universal adaptor oligonucleotide (the linker sequence and universal adaptor oligonucleotide are referred to herein as "LR"), and wherein the LR is attachable to target-specific capture element genetic identifier comprising an LR oligonucleotide via complementary strand interaction. The embodiments of the capture element genetic identifier depicted are oligonucleotides comprising an LR, a template-type element (referred to as "TYPE"), a template ID element (referred to as "ID"), and target-specific capture element (referred to as "Capture region"). The schematic depicts specific template-type elements "A" and "B". The template-type may be any desired nucleic acid sequence as indicated by ellipsis and illustrated by template-type element nucleic acid sequence "n".

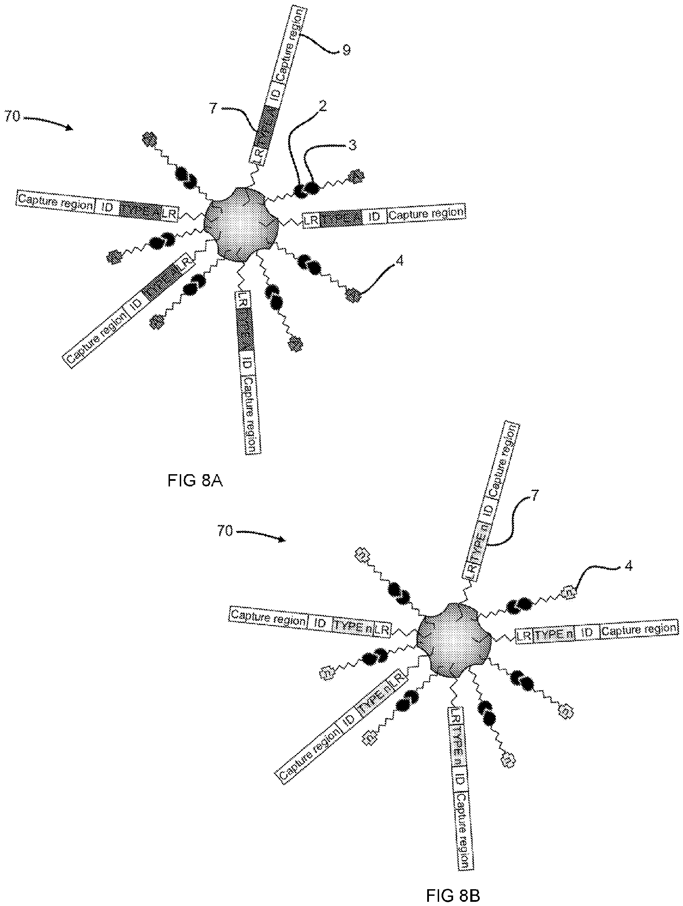

FIGS. 8A-B are schematics depicting two different embodiments of the capture template particle. The capture template particle of FIG. 8A features a capture template particle comprising a capture moiety attached to streptavidin-conjugated target-specific antibody "A", and capture element genetic identifier moiety attached to target-specific capture element genetic identifier comprising a template-type element "A". The capture template particle of FIG.8B depicts a capture template particle comprising a capture moiety attached to streptavidin-conjugated antibody "n", and capture element genetic identifier moiety attached to target-specific capture element genetic identifier comprising a template-type element "n".

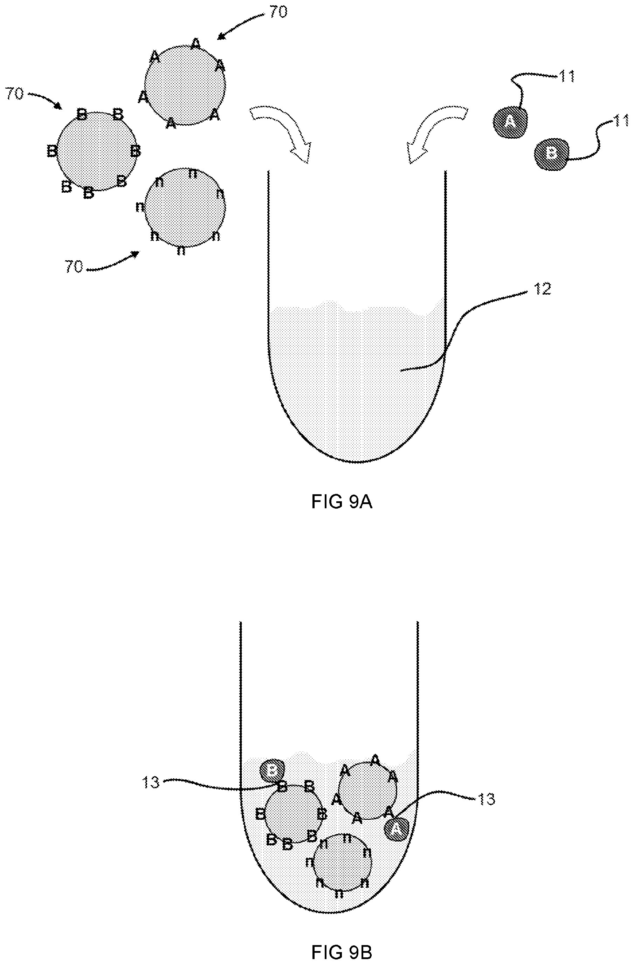

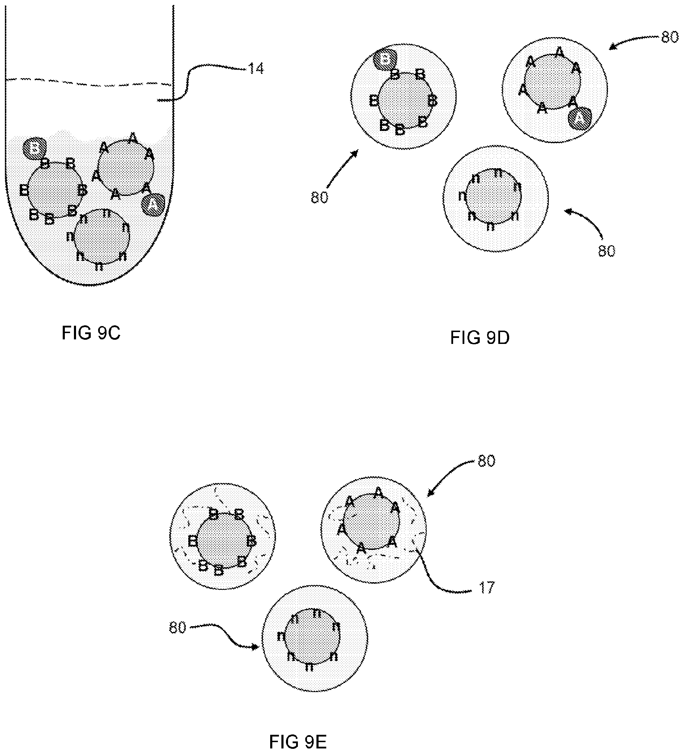

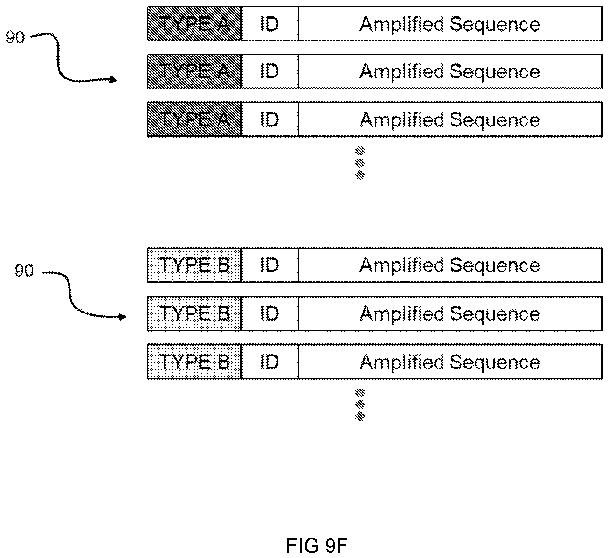

FIGS. 9A-F are schematics generally showing the method of target capture and barcoding according to an embodiment of the present disclosure. Depicted (FIG. 9A) are capture template particles comprising target-specific antibodies (represented schematically by "A", "B", and "n"). The capture template particles are combined (FIG. 9A-B) with target particles (circles marked with A and B) allowing for target-specific capture of a portion of the target particles. The mixture comprising capture template particles and target particles depicted in FIG. 9B is referred to herein as first mixture. Addition of a second fluid (FIG. 9C), such as oil, and shearing are used to generate encapsulations (also referred to herein as "partitions") comprising capture template particles, wherein a portion of the capture template particles is attached to target particles (FIG. 9D). Lysis of the target particles inside the encapsulations releases nucleic acids contained in the template particles (FIG. 9E). Target-specific capture element genetic identifiers (not shown) are used to capture specific portions of the nucleic acids derived from the target particles. The captured nucleic acids can then be amplified. FIG. 9F depicts the amplification products of nucleic acids derived from target particles A and B. As the target-specific capture element genetic identifiers comprise template-type elements which identify the target particle from which the nucleic acids were derived, the amplicons accordingly comprise template-type elements "A" and "B".

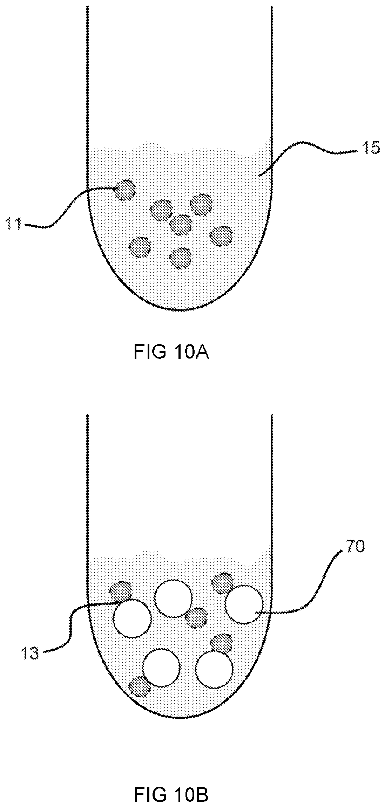

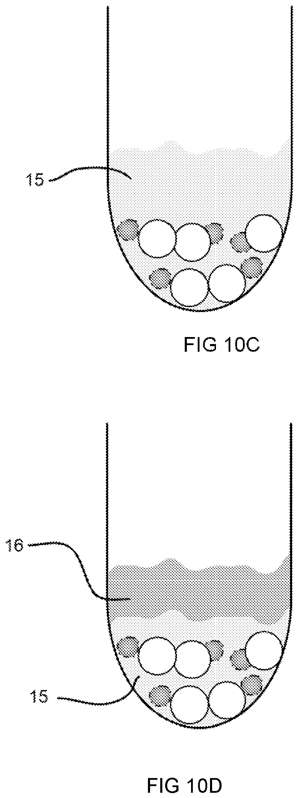

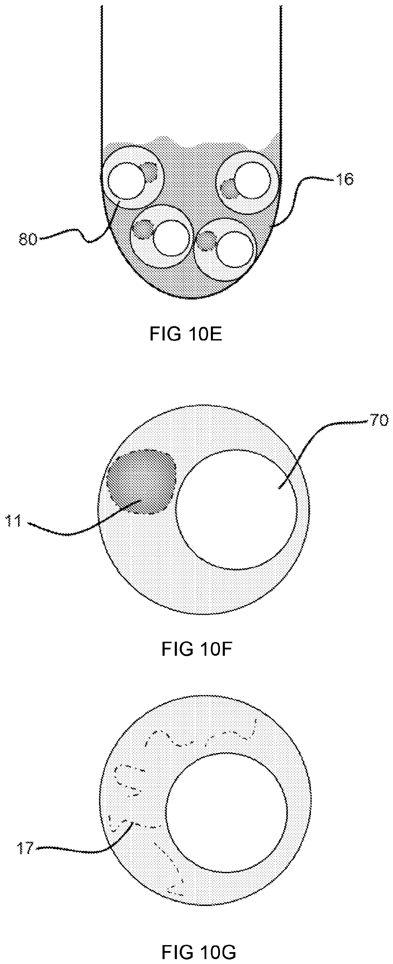

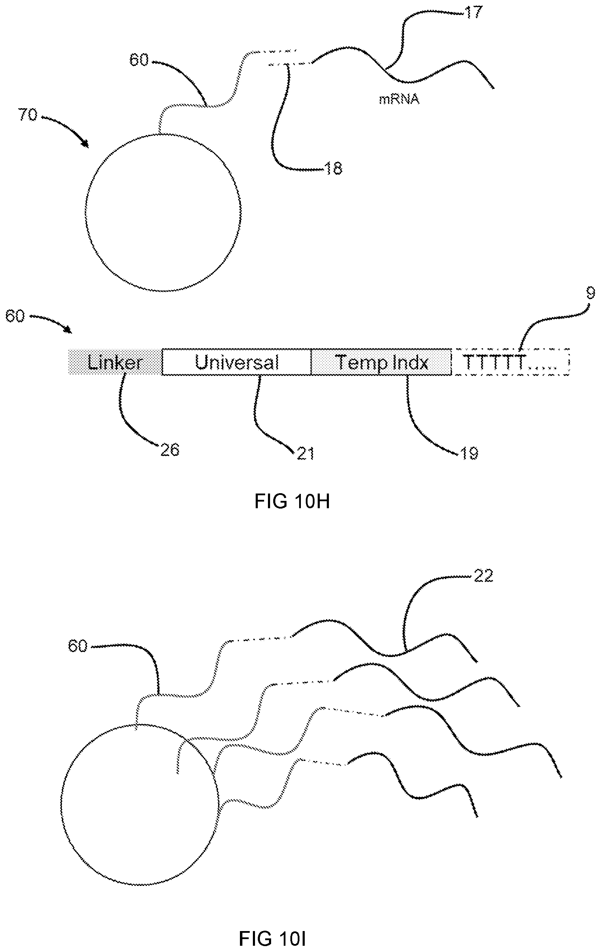

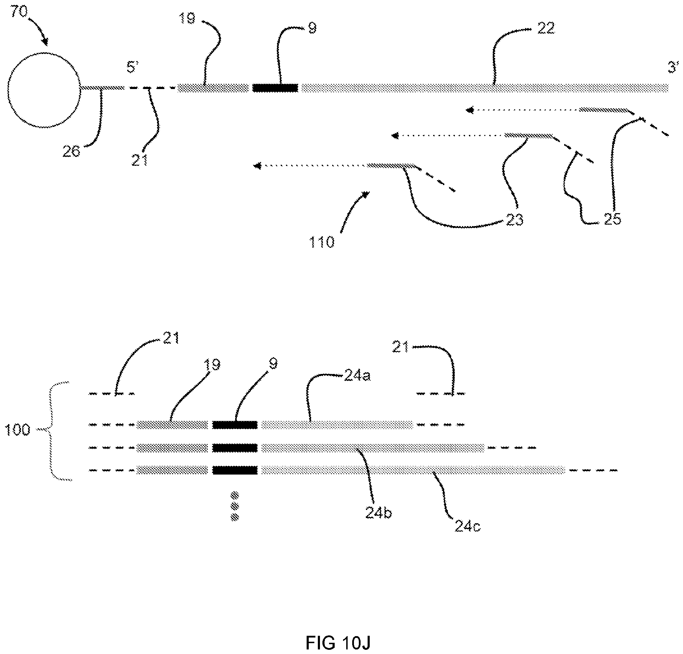

FIG. 10A-J are schematics showing a case workflow of the method of target capture and barcoding in monodisperse droplets according to an embodiment of the present disclosure. A biological sample comprising target particles (FIG. 10A- gray circles) is incubated with capture template particles (white circles) to provide a first mixture (FIG. 10B). Each target-specific association generally comprises one target particle and one capture template particle. The mixture is centrifuged and associated exosomes and target capture particles are pelleted, and the pellet is resuspended in a reaction buffer comprising lysis buffer and rtPCR reagent mix to provide a second mixture (FIG. 10C). Oil ("Partitioning oil", layer depicted in darker gray) is added to the second mixture to provide a third mixture (FIG. 10D). The third mixture is sheared, or partitioned, thereby the capture template particles and associated target particles are encapsulated in a plurality of monodisperse droplets in the partitioning oil. Each capture template particle and associated target particle are encapsulated in a single monodisperse droplet (FIG. 10E). A single encapsulation comprising a capture template particle associated to a target particle is depicted in FIG. 10F. Associated target particles are then lysed to release their contents, including nucleic acids (depicted as broken lines), without breaking the monodisperse droplets encapsulation (FIG. 10G). The capture template particles further comprise a capture element genetic identifier comprising a Poly-T sequence depicted in FIG. 10H. Released mRNA molecules can then may associate with the Poly-T sequences (element bordered with broken line and marked "TTTTT") of the capture template particle's capture element genetic identifier. The other elements of the depicted capture element genetic identifier include a linker sequence ( "Linker") and universal adaptor oligonucleotide ("universal"), and a template index element ( "Temp Indx"). A reverse transcription reaction is then performed to generate cDNA templates of the associated mRNAs. FIG. 10I depicts multiple cDNA molecules attached to the capture template particle's multiple capture element genetic identifiers. An amplification reaction is then performed as illustrated (FIG 10J), wherein hexamer primers (solid gray lines) comprising a universal adaptor sequence "tail" (broken lines) are used to amplify varying length portions of the cDNAs of FIG. 10I. The amplification products of the hexamer primers contain the hexamer's tail comprising a universal adaptor element on their 3' ends, and the Temp Indx and Universal elements, derived from the capture element genetic identifier, on their 5' ends.

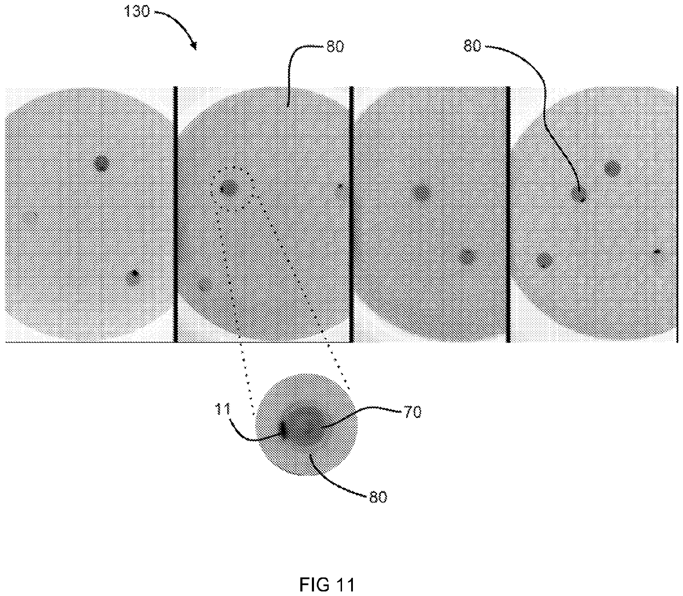

**FIG. 11** is a micrograph of encapsulation generated according to an embodiment of the presently disclosed methods. Some encapsulations comprise a capture template particle. A closeup view is provided of an encapsulation comprising a capture template particle associated with a captured vesicle.

### DETAILED DESCRIPTION OF THE INVENTION

The invention is defined in the appended claims. The present disclosure provides an improved emulsion droplet-based target capture and barcoding method. The present disclosure further provides capture template particles which allow capturing targets of interest from biological samples, and barcoding of specific nucleic acids contained in the captured targets. The nucleic acids can be contained within living or nonliving structures, including particles, viruses, and cells. The nucleic acids can include, e.g., DNA or RNA, which can then be detected, quantitated and/or sorted, e.g., based on their sequence as detected with nucleic acid amplification techniques, e.g., PCR and/or MDA. The disclosed methods involve the use of the capture template particles to template the formation of monodisperse droplets.

Before the present disclosure is further described, it is to be understood that this disclosure is not limited to particular embodiments described, as such may, of course, vary. It is also to be understood that the terminology used herein is for the purpose of describing particular embodiments only, and is not intended to be limiting.

Where a range of values is provided, it is understood that each intervening value, to the tenth of the unit of the lower limit unless the context clearly dictates otherwise, between the upper and lower limit of that range and any other stated or intervening value in that stated range, is encompassed within the present disclosure. The upper and lower limits of these smaller ranges may independently be included in the smaller ranges, and are also encompassed within the present disclosure, subject to any specifically excluded limit in the stated range. Where the stated range includes one or both of the limits, ranges excluding either or both of those included limits are also included in the present disclosure.

Unless defined otherwise, all technical and scientific terms used herein have the same meaning as commonly understood by one of ordinary skill in the art to which this present disclosure belongs. Although any methods and materials similar or equivalent to those described herein can be used in the practice or testing of the present disclosure, some potential and exemplary methods and materials are now described.

It must be noted that as used herein and in the appended claims, the singular forms "a," "an," and "the" include plural referents unless the context clearly dictates otherwise.

Thus, for example, reference to "a droplet" includes a plurality of such droplets and reference to "the nucleic acid" includes reference to one or more nucleic acids and equivalents thereof known to those skilled in the art, and so forth.

It is further noted that the claims may be drafted to exclude any element which may be optional. As such, this statement is intended to serve as antecedent basis for use of such exclusive terminology as "solely", "only" and the like in connection with the recitation of claim elements, or the use of a "negative" limitation.
The publications discussed herein are provided solely for their disclosure prior to the filing date of the present application. Further, the dates of publication provided may be different from the actual publication dates which may need to be independently confirmed.

### Methods

As summarized above, the methods and systems described herein provide capture template particles and an improved emulsion droplet-based target capture and barcoding in monodisperse droplets method thereof. The capture template particles and methods disclosed herein allow capturing targets of interest from biological samples, and barcoding of specific nucleic acids contained in the captured targets. The emulsion droplet-based target capture and barcoding in monodisperse droplets method provides a flexible and sensitive approach to capture targets of interest from biological samples, target and extract specific nucleic acids contained in the captured targets of interest and assign them target-identifying polynucleotide barcodes, amplify the barcoded nucleic acid material, and prepare libraries ready for analysis on sequencing instrumentation.

This presently described but does not claimed capture template particles and an improved emulsion droplet-based target capture and barcoding method thereof leverages the particle-templated emulsification technology previously described. Essentially, micron-scale beads (such as hydrogels) or "template particles" are used to define an isolated fluid volume surrounded by an immiscible partitioning fluid and stabilized by temperature insensitive surfactants.

As used herein, the term "biological sample" encompasses a variety of sample types obtained from a variety of sources, generally the sample types contain biological material. For example, the term includes biological samples obtained from a mammalian subject, e.g., a human subject, and biological samples obtained from a food, water, or other environmental source, etc. The definition encompasses blood and other liquid samples of biological origin, as well as solid tissue samples such as a biopsy specimen or tissue cultures or cells derived therefrom and the progeny thereof. The definition also includes samples that have been manipulated in any way after their procurement, such as by treatment with reagents, solubilization, or enrichment for certain components, such as polynucleotides. The term "biological sample" encompasses a clinical sample, and also includes cells in culture, cell supernatants, cell lysates, cells, serum, plasma, biological fluid, and tissue samples. "Biological sample" includes cells, e.g., bacterial cells or eukaryotic cells; biological fluids such as blood, cerebrospinal fluid, semen, saliva, and the like; bile; bone marrow; skin (e.g., skin biopsy); and antibodies obtained from an individual. Some non-limiting examples of a biological sample include liquid biopsy targets such as circulating cells (tumor, fetal, or stem), cellular components (e.g. nuclei), cell-free nucleic acids, extracellular vesicles, and protein antigens which are being targeted for development of non-invasive diagnostics for a variety of cancers. The term biological sample also includes biological targets indicative of disease such as prokaryotes, fungi, and viruses.

As described more fully herein, in various aspects the subject methods may be used to detect a variety of components from such biological samples. Components of interest include, but are not necessarily limited to, cells (e.g., circulating cells and/or circulating tumor cells), viruses, polynucleotides (e.g., DNA and/or RNA), polypeptides (e.g., peptides and/or proteins), and many other components that may be present in a biological sample.

More specifically, the terms "component of interest", "target of interest", and "target particle" are used interchangeably herein and encompasses any component that may be present in a biological sample.

Polynucleotides" or "oligonucleotides" as used herein refer to linear polymers of nucleotide monomers, and may be used interchangeably. Polynucleotides and oligonucleotides can have any of a variety of structural configurations, e.g., be single stranded, double stranded, or a combination of both, as well as having higher order intra- or intermolecular secondary/tertiary structures, e.g., hairpins, loops, triple stranded regions, etc. Polynucleotides typically range in size from a few monomeric units, e.g. 5- 40, when they are usually referred to as "oligonucleotides," to several thousand monomeric units. Whenever a polynucleotide or oligonucleotide is represented by a sequence of letters (upper or lower case), such as "ATGCCTG," it will be understood that the nucleotides are in 5'→ 3' order from left to right and that "A" denotes deoxyadenosine, "C" denotes deoxycytidine, "G" denotes deoxyguanosine, and "T" denotes deoxythymidine, "I" denotes deoxyinosine, "U" denotes deoxyuridine, unless otherwise indicated or obvious from context. Unless otherwise noted the terminology and atom numbering conventions will follow those disclosed in Strachan and Read, Human Molecular Genetics 2 (Wiley-Liss, New York, 1999).

The terms "polypeptide," "peptide," and "protein," used interchangeably herein, refer to a polymeric form of amino acids of any length. NH2 refers to the free amino group present at the amino terminus of a polypeptide. COOH refers to the free carboxyl group present at the carboxyl terminus of a polypeptide. In keeping with standard polypeptide nomenclature, J. Biol. Chem., 243 (1969), 3552-3559 is used.

In certain aspects, methods are provided for counting and/or genotyping cells, including normal cells or tumor cells, such as CTCs. A feature of such methods is the use of microfluidics.

The methods as described herein generally involve enabling target-specific capture of a plurality of targets of interest by a plurality of capture template particles, and encapsulating the associated target particles and capture template particles in a plurality of monodisperse droplets including monodisperse single-emulsion droplets or multiple- emulsion droplets and/or GUVs, which may be followed by, e.g., one or more nucleic acid synthesis steps, and/or one or more detection and/or sorting steps. The "capture" of the target particle by the capture template particle can be also described herein as binding, attachment, interaction, or otherwise association. Accordingly, the terms "capture", "binding", "attachment", "interaction" and "association" are used interchangeably herein.

### Capture Template Particles and Preparation Thereof

**FIG. 1****,** **FIG. 6, and FIG. 7****.** are schematics depicting the elements of a capture template particle according to an embodiment of the present disclosure. disclosure which is not claimed. In some embodiments, the capture template particle comprises a template particle comprising one or more tethering moieties, wherein each of the one or more tethering moieties is attachable to target-specific elements. The tethering moieties of the present disclosure which are not claimed include capture moiety (FIG. 1-Moiety A, in FIG. 6 element 10), and capture element genetic identifier moiety (FIG. 1- Moiety B, in FIG. 7 element 40). The capture moiety and capture element genetic identifier moiety (40) may comprise a linker which forms part of, and/or is attachable to, the template particle. Various embodiments of the linker and elements attached to it include Acrylate terminated hydrocarbon linker (1), Acrylate-terminated hydrocarbon linker (1) with Biotin termination (2), Acrylate-terminated hydrocarbon linker (1), alkyne-terminated linker (1) with biotin termination (2), or alkyne-terminated linker (1) without biotin termination. In some embodiments, the capture element genetic identifier moiety (FIG. 1- Moiety B, in FIG. 7 element 40) comprises Acrylate terminated hydrocarbon linker (1) with a terminal adaptor polynucleotide (FIG. 1- LR, in FIG. 7 element 6). The template particle is prepared by combining a hydrogel material, e.g., Acrylamide/bisacrylamide copolymer matrix, with one or more of the tethering moieties (10, 40) according to the present disclosure. In some embodiments, the template particle may comprise hydrogel material and the capture moiety (10), in said embodiments, the template particle is prepared by combining hydrogel material, e.g., Acrylamide/bisacrylamide copolymer matrix and Acrylate-terminated hydrocarbon linker with Biotin termination. In other embodiments, the template particle may comprise hydrogel material and the capture element genetic identifier moiety (40), in said embodiments, the template particle is prepared by combining hydrogel material, e.g., Acrylamide/bisacrylamide copolymer matrix and Acrylate terminated hydrocarbon linker with terminal adapter polynucleotide. In yet other embodiments, the template particle comprises hydrogel material, the capture moiety (10), and the capture element genetic identifier moiety (40). Accordingly, in the embodiment wherein the template particle comprises the capture moiety (10) and the capture element genetic identifier moiety (40), it is prepared by combining hydrogel material, e.g., Acrylamide/bisacrylamide copolymer matrix, Acrylate-terminated hydrocarbon linker (1) with Biotin termination (4), and Acrylate terminated hydrocarbon linker (1) with terminal adapter polynucleotide (6). Following the formation of the template particles they are sized to the desired diameter. In some embodiments, sizing of the template particles is done by microfluidic co-flow into an immiscible oil phase. The generation of template particles under microfluidic control is further discussed below.

In some embodiments of the template particles, a variation in diameter or largest dimension of the template particles such that at least 50% or more, e.g., 60% or more, 70% or more, 80% or more, 90% or more, 95% or more, or 99% or more of the template particles vary in diameter or largest dimension by less than a factor of 10, e.g., less than a factor of 5, less than a factor of 4, less than a factor of 3, less than a factor of 2, less than a factor of 1.5, less than a factor of 1.4, less than a factor of 1.3, less than a factor of 1.2, less than a factor of 1.1, less than a factor of 1.05, or less than a factor of 1.01.

As used herein, the term "template particles" and "template particle" are used interchangeably to refer to tiny, generally spherical, particles. Template particles may be porous or nonporous. In any suitable embodiment herein, template particles may include microcompartments, which may contain additional components and/or reagents, e.g., additional components and/or reagents that may be releasable into monodisperse droplets as described herein. In any suitable embodiment herein, template particles may include a polymer, e.g., a hydrogel. In some embodiments, e.g., embodiments as described herein in which the first fluid is an aqueous fluid, the polymer is a hydrophilic polymer. In some embodiments, e.g., embodiments as described herein in which the first fluid is a non-aqueous fluid, e.g., an oil, the polymer is a lipophilic polymer. Template particles generally range from about 0.1 to about 1000 µm in diameter or largest dimension. In some embodiments, template particles have a diameter or largest dimension of about 1.0 µm to 1000 µm, inclusive, such as 1.0 µm to 750 µm, 1.0 µm to 500 µm, 1.0 µm to 250 µm, 1.0 µm to 200 µm, 1.0 µm to 150 µm 1.0 µm to 100 µm, 1.0µm to 10 µm, or 1.0 µm to 5 µm, inclusive. In some embodiments, template particles have a diameter or largest dimension of about 10 µm to about 200 µm, e.g., about 10 µm to about 150 µm, about 10 µm to about 125 µm, or about 10 µm to about 100 µm.

In practicing the methods as described herein which are not claimed, the composition and nature of the template particles may vary. For instance, in certain aspects, the template particles may be microgel particles that are micron-scale spheres of gel matrix. In some embodiments, the microgels are composed of a hydrophilic polymer that is soluble in water, including alginate or agarose. In other embodiments, the microgels are composed of a lipophilic microgel.

The template particles according the invention is a hydrogel. In certain embodiments, the hydrogel is selected from naturally derived materials, synthetically derived materials and combinations thereof. Examples of hydrogels include, but are not limited to, collagen, hyaluronan, chitosan, fibrin, gelatin, alginate, agarose, chondroitin sulfate, polyacrylamide, polyethylene glycol (PEG), polyvinyl alcohol (PVA), acrylamide/bisacrylamide copolymer matrix, polyacrylamide /poly(acrylic acid) (PAA), hydroxyethyl methacrylate (HEMA), poly N-isopropylacrylamide (NIPAM), and polyanhydrides, polypropylene fumarate) (PPF).

In some embodiments, the template particles have an average volume, and a method as described herein includes shrinking the template particles to decrease the average volume. The shrinking may occur upon the application of an external stimulus, e.g., heat. For instance, the template particles may be encapsulated in a fluid by shearing, followed by the application of heat, causing the template particles to shrink in size. The monodisperse single-emulsion droplet or double-emulsion droplet or GUV will not shrink because the droplet volume is constant and dictated by the original size of the template particle, but the template particle within the droplet will shrink away from the surface of the droplet.

In any suitable embodiment herein, the template particles may include at least one of cells, genes, drug molecules, therapeutic agents, particles, bioactive agents, osteogenic agents, osteoconductive agents, osteoinductive agents, anti-inflammatory agents, growth factors, fibroin derived polypeptide particles, nucleic acid synthesis reagents, nucleic acid detection reagents, target particles, DNA molecules, RNA molecules, genomic DNA molecules, and combinations of the same. In embodiments that involve the combination of multiple reagents within a monodisperse single-emulsion droplet or double-emulsion droplet or GUV, the template particles may contain multiple compartments. The template particles may be used to encapsulate reagents that can be triggered to release a desired compound, e.g., a substrate for an enzymatic reaction. For instance, a double emulsion droplet can be encapsulated in the template particles that are triggered to rupture upon the application of a stimulus, e.g., heat. The stimulus initiates a reaction after the template particles have been encapsulated in an immiscible carrier phase fluid.

Template particles may be generated under microfluidic control, e.g., using methods described in U.S. Patent Application Publication No. 2015/0232942. Microfluidic devices can form emulsions consisting of droplets that are extremely uniform in size. The template particles generation process may be accomplished by pumping two immiscible fluids, such as oil and water, into a junction. The junction shape, fluid properties (viscosity, interfacial tension, etc.), and flow rates influence the properties of the template particles generated but, for a relatively wide range of properties, template particles of controlled, uniform size can be generated using methods like T-junctions and flow focusing. To vary template particle size, the flow rates of the immiscible liquids may be varied since, for T-junction and flow focus methodologies over a certain range of properties, template particle size depends on total flow rate and the ratio of the two fluid flow rates. To generate a template particle with microfluidic methods, the two fluids are normally loaded into two inlet reservoirs (e.g., syringes, pressure tubes) and then pressurized as needed to generate the desired flow rates (e.g., using syringe pumps, pressure regulators, gravity, etc.). This pumps the fluids through the device at the desired flow rates, thus generating droplet of the desired size and rate.

In some embodiments, template particles may be generated using parallel droplet generation techniques, including, but not limited to, serial splitting and distribution plates. Parallel droplet generation techniques of interest further include those described by Abate and Weitz, Lab Chip 2011, Jun 7;11(11):1911-5; and Huang et al., RSC Advances 2017, 7, 14932-14938.

In some embodiments, the template particles are allowed to solidify by triggering a gelation mechanism, including, but not limited to, the polymerization or crosslinking of a gel matrix. For instance, polyacrylamide gels are formed by copolymerization of acrylamide and bis-acrylamide. The reaction is a vinyl addition polymerization initiated by a free radicalgenerating system. In certain aspects, agarose hydrogels undergo gelation by cooling the hydrogels below the gelation temperature.

In some embodiments, the template particles may be removed from the fluid, dried, and stored in a stable form for a period of time. Examples of drying approaches include, but are not limited to, heating, drying under vacuum, freeze drying, and supercritical drying. In some embodiments, the dried template particles may be combined with a fluid, but still retain the shape and structure as independent, often spherical, gel particles. In some embodiments, the dried template particles are combined with an appropriate fluid, causing a portion of the fluid to be absorbed by the template particles. In some embodiments, the porosity of the template particles may vary, to allow at least one of a plurality of target particles to be absorbed into the template particles when combined with the appropriate fluid. Any convenient fluid that allows for the desired absorption to be performed in the template particles may be used.

As used herein, the terms "absorb," "swell," and "expand" as applied to template particles may be used interchangeably to refer to the process in which a fluid permeates a substance, or in which a substance incorporates a fluid. In some embodiments, the substance being absorbed may retain at least a portion of its shape and structure. In some embodiments, the substance being absorbed may become incorporated into a fluid so as to form a solution.

In certain aspects, a surfactant may be used to stabilize the template particles. Accordingly, a template particle may involve a surfactant stabilized emulsion, e.g., a surfactant stabilized single emulsion or a surfactant stabilized double emulsion. Any convenient surfactant that allows for the desired reactions to be performed in the template particles may be used. In other aspects, a template particle is not stabilized by surfactants or particles.

To generate the capture template particles, target-specific elements are attached to the sized template particles. The target-specific elements of the present disclosure are selected from target-specific capture elements, and target-specific capture element genetic identifier. The target-specific capture elements comprise Poly-T polynucleotide sequences, aptamers, and antibodies. In some embodiments, the target-specific capture elements comprise streptavidin (3), and may therefore attach to the capture moiety by biotin-streptavidin affinity. A schematic representation of target-specific (the type of antibody represented by different shades of gray and are labeled in FIG. 1, and by letters "A", "B" etc. to "n" in FIG. 6) streptavidin conjugated antibodies is shown (20). In some embodiments, the antibodies (4) are conjugated to the streptavidin (3) via a hydrocarbon linker (5).

The target-specific capture element genetic identifier (FIG. 1, and in FIG. 7 element 50) is an oligonucleotide and comprises elements selected from an LR oligonucleotide (in FIG7 element 6), a template-type element (also referred to herein as "TYPE", and in FIG. 7 element 7), a template ID element (also referred to herein as "ID", and in FIG. 7 element 8), and target-specific capture element (also referred to herein as "Capture region", and in FIG. 7 element 9), and combinations thereof. In some embodiments, the target-specific capture element genetic identifier (50), comprising an LR sequence (6) complementary to the LR sequence of the capture element genetic identifier moiety, can attach to the capture element genetic identifier moiety (40) via complementary strand interaction between the LR sequences (6).

Attachment of the target-specific elements (20, 50) to the template particles occur by incubating said target-specific elements (20, 50) with template particles. Incubation may be for the amount of time, temperature, and otherwise conditions sufficient to allow the target-specific elements (20, 50) to attach to the template particles. In some embodiments, incubation comprises shaking, mixing, flowing, or agitation in order to increase the exposure of the target-specific elements to the template particles.

The target-specific capture element (50) comprises a polynucleotide complementary to a section on the target nucleic acid (in FIG. 7 element 9, also referred to herein as the "target-specific capture element" or "capture region") and is used to capture the said nucleic acid target.

In some embodiments, the target-specific capture element genetic identifier (50) corresponds to the target-specific capture element (20) present in/on the same capture template particle, i.e., it barcodes the type of target-specific capture element (20) used (said barcode is depicted in FIG. 1 as "TYPE", and in FIG. 7 element 7). Illustrated schematically, when a capture template particle comprises a specific antibody-Antibody 1, the target-specific capture element genetic identifier attached to the same capture template particle will have a certain barcode sequence which corresponds only to Antibody 1. This embodiment is also illustrated in FIGs. 8A-B which are further discussed below. In addition, the ID sequences (8) of the target-specific capture element genetic identifier (50) are another barcode which can be traced to the capture template particle to which they are attached. Thus, the target-identifying polynucleotide barcodes, TYPE (7) and ID (8), enable tracing the origin of the nucleic acid amplified using the emulsion droplet-based target capture and barcoding method.

The "barcode" or "barcode sequence" as referred to herein, are polynucleotide sequences which are unique, i.e., distinguishable from other barcode sequences. The sequences may be of any suitable length which is sufficient to distinguish the barcode sequence from other barcode sequences. A barcode sequence may have a length of 5, 6, 7, 8, 9, 10, 11, 12, 13, 14, 15, 16, 17, 18, 19, 20, 21, 22, 23, 24, 25 nucleotides, or more. In some embodiments, the barcodes are predefined and selected at random.

Embodiments of the presently disclosed capture template particle are depicted in FIGS. 8A-B. The capture template particle of FIG.8A (70) comprises a capture moiety attached to streptavidin-conjugated antibody "A" (30), and a capture element genetic identifier moiety attached to target-specific capture element genetic identifier comprising a template-type element "A" (60). Therefore, the origin of the nucleic acids captured by the target-specific capture element genetic identifier can be traced to the captured target particle from which they were derived. FIG. 8B exemplifies this embodiment wherein a capture template particle comprises a capture moiety attached to streptavidin-conjugated antibody "n" (30), and also a capture element genetic identifier moiety attached to target-specific capture element genetic identifier comprising a template-type element "n" (60). Thus the template type corresponds to, and identifies, the antibody used.

### Capture Template Particles and Emulsion Droplet Based Target Capture and Barcoding Method Thereof

**FIG. 2** is a schematic generally showing the workflow of the method of target capture and barcoding in monodisperse droplets according to an embodiment of the present disclosure. Capture template particles comprising target-specific capture elements and target-specific capture element genetic identifier elements are represented schematically by gray circles surrounded by crosses, wherein the crosses represent the target-specific capture elements. The capture template particles are combined with target particles (depicted as small circles). The mixture of capture template particle and target particles is incubated for a sufficient amount of time to allow target-specific association of the target particles with the capture elements. In some embodiments, agitation or mixing is used to increase the probability of said target-specific association. To form encapsulations (also referred to herein as "partitions"), the mixture comprising the bound target particles and capture template particles is combined with a second fluid to provide a new mixture, wherein the second fluid is immiscible with the said mixture comprising the bound target particles and capture template particles. In some embodiments the second fluid is an oil. The next step includes shearing the new mixture such that a plurality of monodisperse droplets is formed. In some embodiments, a portion of the monodisperse droplets comprise a capture template particle. In some embodiments, each capture template particle, whether or not associated with a target particle, is consequently encapsulated in monodisperse droplets (depicted by the larger gray circles). Associated target particles are then lysed within the encapsulation to release nucleic acids (depicted by black lines within the monodisperse droplet). Released nucleic acids are sufficiently free from any previous compartmentalization to be able to interact with the elements of the capture template particle.

Lysis of the target particles within the encapsulation may be done using any method known in the art. In some embodiments, the lysis method is selected from high temperature degradation, high temperature lysis reagent release from within the hydrogel matrix of the capture template particle, rapid co-partitioning, or lysis with active lysis buffer. Lysis of captured target particles is further discussed below.

Upon the release of nucleic acids from the lysed target particle, a selected portion of the nucleic acids may then associate, via complementary strand interaction, with the target-specific capture element genetic identifier's capture region sequence. Associated nucleic acids can then be amplified using primers specific to the target nucleic acid sequence and the LR sequence of the target-specific capture element genetic identifier. The amplification products comprise the ID and TYPE sequences of the target-specific capture element genetic identifier and can be traced using these barcodes to the target-specific capture element used to capture the target particle from which they originated, and to the single capture template particles to which said target-specific capture element was attached. In any of the applications of the presently disclosed target capture and barcoding method, a sequencing library may be prepared from the amplification products using methods standard in the art.

Similarly, FIGS. 9A-F are schematics generally showing the workflow of the method of target capture and barcoding in monodisperse droplets according to an embodiment of the present disclosure. The capture template particles (70) comprise target-specific capture elements and target-specific capture element genetic identifier elements, wherein different portions of the capture template particles (70) comprise different antibodies. In this example, a portion of the capture template particles comprises specific antibody "A", while another portion comprises antibody "B", and yet another portion comprises antibody "n". Therefore, the capture template particles of the example of FIGS. 9A-F are designed to capture three different specific target particles. The antibodies represented by "A", "B", or "n" may be any antibody suitable for use in connection with the presently described methods. The capture template particles (70) are combined with target particles (11). The mixture of capture template particle and target particles is incubated for a sufficient amount of time to allow target-specific association of the target particles with the capture elements. In some embodiments, agitation or mixing is used to increase the probability of said target-specific association. To form encapsulations (also referred to as "partitions"), the mixture comprising the bound target particles and capture template particles is combined with a second fluid (14) to provide a new mixture, wherein the second fluid is immiscible with the mixture comprising the bound target particles and capture template particles. In some embodiments, the buffer (12) in which the capture template particles (70) and target particles (11) are combined comprises lysis reagent/s and/or rtPCR reagent mix. In some embodiments the second fluid is an oil. The next step includes shearing the new mixture such that a plurality of monodisperse droplets is formed. In some embodiments, a portion of the monodisperse droplets comprise a capture template particle (80). In some embodiments, each capture template particle, whether or not associated with a target particle, is consequently encapsulated in monodisperse droplets. Associated target particles are then lysed (FIG. 9E) within the encapsulation to release nucleic acids (17). Released nucleic acids are sufficiently free from any previous compartmentalization to be able to interact with the elements of the capture template particle (not shown). Lysis is performed using any method known in the art and described herein.

Upon the release of nucleic acids from the lysed target particle, a selected portion of the nucleic acids may then associate, via complementary strand interaction, with the target-specific capture element genetic identifier's capture region sequence. Associated nucleic acids can then be amplified using, for example, primers specific to the target nucleic acid sequence and the LR sequence of the target-specific capture element genetic identifier. Each amplification product (90) comprises the specific ID and TYPE sequences of the target-specific capture element genetic identifier and can be traced using these barcodes to the target-specific capture element which was used to capture the target particle from which they were derived, and to the single capture template particles to which said target-specific capture element was attached. In any of the applications of the presently disclosed target capture and barcoding method, a sequencing library may be prepared from the amplification products using methods standard in the art.

The schematics of FIG. 10A-J further elaborate of an embodiment of the method of target capture and barcoding. The example features a biological sample comprising target particles (e.g. exosome vesicles) (11) which are incubated with capture template particles (also referred to herein as "capture PIPS", 70) to provide a first mixture. The capture template particles further comprise a capture element genetic identifier comprising a Poly-T sequence (not shown). In some embodiments, each association comprises one exosome and one capture template particle. In the next step, the first mixture is centrifuged and associated exosomes and target capture particles are pelleted. The supernatant is discarded (not shown) and the pellet is resuspended in a buffer (15) which may comprise lysis reagent/s and reverse transcription PCR reagents mix to provide a second mixture. The centrifugation step, as described above, may increase the proportion of the capture template particles bound to a target particle (this increase is also referred to herein as "enrichment"). It also allows the addition of the lysis reagent/s and reverse transcription reagents mix to facilitate the generation of cDNA within the encapsulations. Other methods of enrichment are discussed below. A second fluid generally immiscible with the second mixture, such as an oil ("Partitioning oil", 16) is then added to the second mixture to provide a third mixture. In some embodiments, the centrifugation and resuspension steps are omitted, and the first mixture is combined, following the incubation step as described herein, with the second fluid (16) to enable the shearing step described below. In the embodiment that the centrifugation and resuspension steps are omitted, lysis reagent/s and/or rtPCR reagent mix may be present in the incubation buffer (FIG. 10A, 15), alternatively they may be added to the first mixture following the incubation step, or in some other embodiments they may be present in the second fluid. Addition of lysis reagent/s via the second fluid is further discussed below.

The next step comprises shearing (also referred to herein as "partitioning") the third mixture thereby the encapsulating the capture template particles and associated exosomes in a plurality of monodisperse droplets in the partitioning oil (FIG. 10E, 80). In some embodiments, each capture template particle and associated exosome are encapsulated in a single monodisperse droplet. A micrograph of a target capture particle (70) associated with a vesicle (11) and encapsulated in a monodisperse droplet (80) is shown in FIG. 11. In the embodiment in which the resuspension buffer of the step of FIG. 10C comprised lysis agent/s and reverse transcription reagents mix, each monodisperse droplet contains the lysis agent/s and reverse transcription PCR reagents mix. Associated target particles are then lysed within the monodisperse droplets using any suitable lysis method and subsequently release their contents, including nucleic acids (17). Released mRNA molecules may then associate, via their poly A tails (18), with the Poly-T sequences (9) of the capture template particle's capture element genetic identifier (50). The other elements of the depicted capture element genetic identifier (50) include an LR oligonucleotide comprising a linker sequence and universal adaptor oligonucleotide (26, 21), and a template index element (19). The template index element (19) may comprise such elements as the TYPE element and/or ID element previously described, or indeed any sequence usable for tracing the origin of the associated nucleic acids to a particular target particle. In some embodiments, multiple mRNA molecules may be attached to the capture template particle's multiple capture element genetic identifiers.

Following the binding of the released mRNA molecules to the Poly-T sequences, reverse transcription reaction is performed to generate cDNA templates of the associated mRNAs. FIG. 10I depicts the cDNAs generated (22) attached to the capture template particle via the capture element genetic identifier moieties attached to target-specific capture element genetic identifiers (60). Amplification of the cDNA (22) can be performed using primers which comprise sequences specific to a desired target cDNA and thereby will amplify a specific pre-determined portion of the sequence of the said cDNA. Alternatively, amplification of the associated cDNAs (22) can be done using random priming, thereby creating a library of random fragment (portion of nucleic acid) length representing various portions of the associated cDNA sequences. The present example depicts random priming using hexamer primers (110), comprising a random six nucleotide sequence (23) and a "tail" (25) comprising a universal adaptor sequence (21), to create amplicons of random fragment length. The amplification products of the hexamer primers (100) comprise the hexamer's tail (25) comprising the universal adaptor element (21) on their 3' ends, and the universal adaptor element (21), temp index element (19), and capture region (9) derived from the capture element genetic identifier (50), on their 5' ends. All fragments from same exosome carry the same temp index element (19). Universal amplification can be subsequently performed to create a library of random fragment length (24a-c).

**FIG. 3-5** schematically depict a case workflow of the method of target capture and barcoding according to an embodiment of the present disclosure. The example features a biological sample comprising exosome vesicles (gray circles) which are incubated with capture template particles (also referred to herein as "capture PIPS") to provide a first mixture. In the embodiment depicted, the target-specific capture elements comprise anti-Tetraspanin antibodies. Tetraspanins are transmembrane proteins abundant in exosomes, and therefore a suitable target for capture by an anti-Tetraspanin antibody target-specific capture element. The capture template particles further comprise a capture element genetic identifier comprising a Poly-T sequence. In some embodiments, each association comprises one exosome and one capture template particle. In the next step, the first mixture is centrifuged and associated exosomes and target capture particles are pelleted. The supernatant is discarded (not shown) and the pellet is resuspended in a reaction buffer comprising lysis buffer and reverse transcription PCR reagents mix to provide a second mixture. The centrifugation step, as described above, may increase the proportion of the capture template particles bound to a target particle (this increase is also referred to herein as "enrichment"). It also allows the addition of the lysis buffer and reverse transcription reagents mix to facilitate generation of cDNA within the encapsulations. Other methods of enrichment are discussed below. Oil ("Partitioning oil"), a fluid which is immiscible with the second mixture, is added to the second mixture to provide a third mixture (illustrated in FIG. 4).

The next step comprises shearing (also referred to herein as "partitioning") the third mixture such that the capture template particles and associated exosomes are encapsulated in a plurality of monodisperse droplets in the partitioning oil. In some embodiments, each capture template particle and associated exosome are encapsulated in a single monodisperse droplet. In addition, each monodisperse droplet contains the lysis buffer and reverse transcription PCR reagents mix. Associated exosomes are then lysed within the monodisperse droplets using temperature activated lysis to release their contents, including nucleic acids (depicted as black lines). Released mRNA molecules may then associate with the Poly-T sequences ("Poly-T capture") of the capture template particle's capture element genetic identifier. The other elements of the depicted capture element genetic identifier include an LR oligonucleotide comprising a linker sequence (also referred to herein as "Linker", depicted in dark gray) and universal adaptor oligonucleotide (also referred to herein as "universal PCR adaptor", depicted in red), and a template ID element (also referred to herein as "Template specific barcode"). In some embodiments, multiple mRNA molecules may be attached to the capture template particle's multiple capture element genetic identifiers.

Following the binding of the released mRNA molecules to the Poly-T sequences, reverse transcription reaction is performed to generate cDNA templates of the associated mRNAs. A DNA reverse transcription reaction then takes place, using hexamer primers (also referred to herein as "Hexamer primers - (in bulk)") comprising a universal adaptor sequence "tail" (also referred to herein as "Universal PCR adaptor") to create a library of random fragment length. The amplification products of the hexamer primers comprise the hexamer's tail comprising the universal adaptor element on their 3' ends, and the ID, TYPE, and Universal elements, derived from the capture element genetic identifier, on their 5' ends. All fragments from same exosome carry the same template ID element (also referred to herein as "barcode")., If desired, a sequencing library is prepared from the PCR amplification products using standard methods.

### PCR Methods

A feature of certain methods as described herein is the use of a polymerase chain reaction (PCR)-based assay to detect the presence of certain oligonucleotides and/or genes, e.g., oncogene(s) present in cells. Examples of PCR-based assays of interest include, but are not limited to, quantitative PCR (qPCR), quantitative fluorescent PCR (QF-PCR), multiplex fluorescent PCR (MF-PCR), digital droplet PCR (ddPCR) single cell PCR, PCR-RFLP/real time-PCR-RFLP, hot start PCR, nested PCR, in situ polony PCR, in situ rolling circle amplification (RCA), bridge PCR, picotiter PCR, emulsion PCR and reverse transcriptase PCR (RT-PCR). Other suitable amplification methods include the ligase chain reaction (LCR), transcription amplification, self-sustained sequence replication, selective amplification of target polynucleotide sequences, consensus sequence primed polymerase chain reaction (CP-PCR), arbitrarily primed polymerase chain reaction (AP-PCR), degenerate oligonucleotide-primed PCR (DOP- PCR) and nucleic acid based sequence amplification (NABSA).

A PCR-based assay may be used to detect the presence of certain gene(s), such as certain oncogene(s). In such assays, one or more primers specific to each gene of interest are reacted with the genome of each cell. These primers have sequences specific to the particular gene, so that they will only hybridize and initiate PCR when they are complementary to the genome of the cell. If the gene of interest is present and the primer is a match, many copies of the gene are created. To determine whether a particular gene is present, the PCR products may be detected through an assay probing the liquid of the monodisperse droplet, such as by staining the solution with an intercalating dye, like SybrGreen or ethidium bromide, hybridizing the PCR products to a solid substrate, such as a bead (e.g., magnetic or fluorescent beads, such as Luminex beads), or detecting them through an intermolecular reaction, such as FRET. These dyes, beads, and the like are each example of a "detection component," a term that is used broadly and generically herein to refer to any component that is used to detect the presence or absence of nucleic acid amplification products, e.g., PCR products.

### Monodisperse Droplets, Including Single-Emulsion Droplets and Multiple-Emulsion Droplets, and Generation Thereof

Monodisperse droplets may be effectively obtained by using capture particles to template the formation of droplets, which can include, e.g., monodisperse single-emulsion droplets, multiple- emulsion droplets, or Giant Unilamellar Vesicles (GUV)

As used herein, the term "monodisperse," as applied to droplets, e.g., monodisperse single-emulsion droplets, refers to a variation in diameter or largest dimension of droplets produced by shearing in the presence of capture template particles, which is less than would occur when droplets are produced by shearing under the same conditions in the absence of the capture template particles. Generally, monodisperse single-emulsion droplets or multiple-emulsion droplets can have more variation in diameter or largest dimension as compared to the capture template particles from which they are generated, while still functioning in the various methods described herein. Monodisperse droplets generally range from about 0.1 to about 1000µm in diameter or largest dimension, and may have a variation in diameter or largest dimension of less than a factor of 10, e.g., less than a factor of 5, less than a factor of 4, less than a factor of 3, less than a factor of 2, less than a factor of 1.5, less than a factor of 1.4, less than a factor of 1.3, less than a factor of 1.2, less than a factor of 1.1, less than a factor of 1.05, or less than a factor of 1.01, in diameter or the largest dimension. In some embodiments, monodisperse droplets have a variation in diameter or largest dimension such that at least 50% or more, e.g., 60% or more, 70% or more, 80% or more, 90% or more, 95% or more, or 99% or more of the monodisperse droplets, vary in diameter or largest dimension by less than a factor of 10, e.g., less than a factor of 5, less than a factor of 4, less than a factor of 3, less than a factor of 2, less than a factor of 1.5, less than a factor of 1.4, less than a factor of 1.3, less than a factor of 1.2, less than a factor of 1.1, less than a factor of 1.05, or less than a factor of 1.01. In some embodiments, monodisperse droplets have a diameter of about 1.0 µm to 1000 µm, inclusive, such as about 1.0 µm to about 750 µm, about 1.0 µm to about 500 µm, about 1.0 µm to about 250 µm, about 1.0 µm to about 200 µm, about 1.0 µm to about 150 µm, about 1.0 µm to about 100 µm, about 1.0 µm to about 10 µm, or about 1.0 µm to about 5 µm, inclusive. In some embodiments, the internal volume of the monodisperse droplets may be about 0.01 pL or less, about 0.1 pL or less, 1 pL or less, about 5 pL or less, 10 pL or less, 100 pL or less, or 1000 pL or less. In some embodiments, the internal volume of the monodisperse droplets may be about 1 fL or less, about 10fL or less, or 100 fL or less. In some embodiments, the internal volume of the monodisperse droplets may encompass a liquid volume which ranges between picoliters and femotliters (e.g., about 0.001 pL to about 1000 pL). In some embodiments, the internal volume of the monodisperse droplets extends strictly below the nanoliter level (e.g., strictly picoliter, strictly femtoliter, or combination thereof).

In practicing the methods as described herein, the composition and nature of the monodisperse droplets, e.g., single-emulsion and multiple-emulsion droplets, may vary. For instance, in certain aspects, a surfactant may be used to stabilize the droplets.

Accordingly, a droplet may involve a surfactant stabilized emulsion, e.g., a surfactant stabilized single emulsion or a surfactant stabilized double emulsion. Any convenient surfactant that allows for the desired reactions to be performed in the droplets may be used. In other aspects, monodisperse droplets are not stabilized by surfactants.

The droplets described herein may be prepared as emulsions, e.g., as an aqueous phase fluid dispersed in an immiscible phase carrier fluid (e.g., a fluorocarbon oil, silicone oil, or a hydrocarbon oil) or vice versa. In particular, multiple-emulsion droplets as described herein may be provided as double-emulsions, e.g., as an aqueous phase fluid in an immiscible phase fluid, dispersed in an aqueous phase carrier fluid; quadruple emulsions, e.g., an aqueous phase fluid in an immiscible phase fluid, in an aqueous phase fluid, in an immiscible phase fluid, dispersed in an aqueous phase carrier fluid; and so on. Generating a monodisperse single-emulsion droplet or a multiple-emulsion droplet as described herein may be performed without microfluidic control. In alternative embodiments, a monodisperse single-emulsion may be prepared without the use of a microfluidic device, but then modified using a microfluidic device to provide a multiple emulsion, e.g., a double emulsion.

Monodisperse single emulsions may be generated without the use of microfluidic devices using the methods described herein. Producing a monodisperse emulsion using capture template particles can provide emulsions including droplets that are extremely uniform in size. The droplet generation process may be accomplished by combining a plurality of capture template particles with a first fluid to provide a first mixture, wherein the first fluid includes a plurality of target particles; combining the first mixture with a second fluid to provide a second mixture, wherein the second fluid is immiscible with the first fluid; and shearing the second mixture such that a plurality of the capture template particles are encapsulated in a plurality of monodisperse droplets in the second fluid, thereby providing a plurality of monodisperse droplets including the first fluid, one of the capture template particles, and one of the plurality of target particles. To vary droplet size, the shearing rate and capture template particle sizes may be varied. For agarose gels, the capture template particles can be liquefied using an external stimulus (e.g., heat) to generate a liquid monodisperse emulsion.

The percentage of monodisperse droplets, e.g., monodisperse single-emulsion droplets or multiple-emulsion droplets, with one, and not more than one, capture template particle may be about 70% or more; about 75% or more; about 80% or more; about 85% or more; about 90% or more; or about 95% or more. For example, the percentage of monodisperse droplets with one, and not more than one, capture template particle may be from about 70% to about 100%, e.g., from about 75% to about 100%, from about 80% to about 100%, from about 85% to about 100%, from about 90% to about 100%, or from about 95% to about 100%. As a further example, the percentage of monodisperse droplets with one, and not more than one, capture template particle may be from about 70% to about 95%, e.g., from about 75% to about 90%, or from about 80% to about 85%. The percentage of capture template particles that are encapsulated in monodisperse droplets in the second fluid may be about 70% or more; about 75% or; about 80% or more; about 85% or more; or about 90% or more. For example, the percentage of capture template particles that are encapsulated in monodisperse droplets in the second fluid may be from about 70% to about 100%, e.g., from about 75% to about 100%, from about 80% to about 100%, from about 85% to about 100%, from about 90% to about 100%, or from about 95% to about 100%. As a further example, the percentage of capture template particles that are encapsulated in monodisperse droplets in the second fluid may be from about 70% to about 95%, e.g., from about 75% to about 90%, or from about 80% to about 85%.

Double emulsions may also be generated without the use of microfluidic devices using the methods described herein. A double emulsion includes droplets contained within droplets, e.g., an aqueous phase fluid surrounded by an immiscible phase shell in an aqueous phase carrier fluid (e.g., water-in oil-in water) or a immiscible phase fluid surrounded by an aqueous phase shell in an immiscible phase carrier fluid (e.g., oil-in water-in oil). The second mixture described herein, which includes monodisperse single- emulsion droplets in the second fluid, is combined with a third fluid to produce a third mixture, wherein the third fluid is immiscible with at least the second fluid. The third mixture is then sheared to encapsulate the capture template particles in double- emulsion droplets in the third fluid. The third fluid may be immiscible with both the first and second fluids. A particularly useful kind of double emulsion includes an aqueous droplet encapsulated within a slightly larger oil droplet, itself dispersed in a carrier aqueous phase. Double emulsions are valuable because the inner "core" of the structure can be used to provide active compounds, like dissolved solutes or biological materials, where they are shielded from the external environment by the surrounding oil shell. A benefit of generating double emulsions using capture template particles is similar to that for the generation of single emulsions, in that the double emulsion dimensions (inner and outer droplet sizes) can be controlled over a wide range and the droplets can be formed with a high degree of uniformity. As discussed herein, in suitable embodiments the capture template particles can be dissolved and/or melted within the monodisperse droplets. Accordingly, in some embodiments multiple emulsions, e.g., double emulsions, may be prepared from monodisperse droplets which no longer contain an intact template particle yet retain their original size. In this manner, such monodisperse droplets may serve as templates for the preparation of multiple emulsions, e.g., double emulsions.

Encapsulation in droplets of sample materials and/or reagents, e.g., nucleic acids and/or nucleic acid synthesis reagents (e.g., isothermal nucleic acid amplification reagents and/or nucleic acid amplification reagents), can be achieved via a number of methods, including microfluidic and non-microfluidic methods. In the context of microfluidic methods, there are a number of techniques that can be applied, including glass microcapillary double emulsification or double emulsification using sequential droplet generation in wettability patterned devices. Microcapillary techniques form droplets by generating coaxial jets of the immiscible phases that are induced to break into droplets via coaxial flow focusing through a nozzle. However, a potential disadvantage of this approach is that the devices are generally fabricated from microcapillary tubes that are aligned and glued together. Since the drop formation nozzle is on the scale of tens of microns, even small inaccuracies in the alignment of the capillaries can lead to a device failure. By contrast, sequential drop formation in spatially patterned droplet generation junctions can be achieved in devices fabricated lithographically, making them simpler to build and to create in large numbers while maintaining uniformity over dimensions.

However, in some cases the planar nature of these devices may not be ideal for generating double emulsions, since the separate phases all enter the device while in contact with the channel walls, necessitating that wettability be carefully patterned to enable engulfment of the appropriate phases at the appropriate locations. This may make the devices more difficult to fabricate, and in some cases, may prevent emulsification of liquids whose wetting properties are not optimized for the device. Accordingly, in some aspects the present disclosure provides methods for generating a monodisperse emulsion which encapsulates sample materials and/or reagents, e.g., nucleic acids and/or nucleic acid synthesis reagents (e.g., isothermal nucleic acid amplification reagents and/or nucleic acid amplification reagents) without the use of a microfluidic device.

For example, the methods as described herein may include combining a plurality of capture template particles with a first fluid to provide a first mixture, wherein the first fluid includes a plurality of target particles, e.g., nucleic acids, etc. In some embodiments, the combining the plurality of capture template particles with the first fluid to provide the first mixture includes causing a portion of the first fluid, and the target particles and/or reagents contained therein, to be absorbed, or attached, by the capture template particles. In some embodiments, combining the plurality of capture template particles with the first fluid to provide the first mixture includes flowing a portion of the first fluid into the capture template particles. In some embodiments, combining the plurality of capture template particles with the first fluid to provide the first mixture includes diffusing a portion of the first fluid into the capture template particles. In some embodiments, the combining the plurality of capture template particles with the first fluid to provide the first mixture includes swelling the capture template particles with a portion of the first fluid.

In some embodiments, excess first fluid is removed from the first mixture after causing the portion of the first fluid to be absorbed by the capture template particles. The amount of excess first fluid removed may vary. For example, by removing most of the excess fluid, target particles that cannot flow into the capture template particles can be encapsulated by causing target particles, e.g., cells, to be physically close to at least one of a plurality of capture template particles. Combining this mixture with a oil which is immiscible with the first fluid provides a second mixture. By shearing this second mixture, a plurality of the capture template particles may be encapsulated in a plurality of monodisperse droplets in the second fluid, thereby providing a plurality of monodisperse droplets including the first fluid, one of the capture template particles, and one of the plurality of target particles that cannot flow into the capture template particles.

In some embodiments, the target molecules are cells. In such embodiments, the monodisperse droplets may attach one or more cells per droplet. Alternatively, the monodisperse droplets do not contain more than one cell per droplet. In some embodiments, after shearing, some droplets in the emulsion do not contain any of the plurality of target particles.

In some embodiments, the methods disclosed herein include combining the first mixture, including a plurality of capture template particles and a first fluid including a plurality of target particles, with a second fluid to provide a second mixture, wherein the second fluid is immiscible with the first fluid; and shearing the second mixture such that a plurality of the capture template particles are encapsulated in a plurality of monodisperse droplets in the second fluid, thereby providing a plurality of monodisperse droplets including the first fluid, one of the capture template particles, and one of the plurality of target particles. In some embodiments, after shearing, the second fluid includes a plurality of droplets that do not contain one of the capture template particles. The droplets that do not contain one of the capture template particles may be removed from the monodisperse emulsion by a suitable separation technique, such as filtration or centrifugation. Those droplets that do not contain one of the capture template particles may be smaller in diameter than those droplets that do contain one of the capture template particles. The monodisperse droplets containing capture template particles may also be enriched relative to droplets that do not contain one of the capture template particles.

As used herein, the terms "enriched" and "enrichment" may be used interchangeably to refer to the process of increasing the ratio of target entities (e.g., monodisperse droplets containing capture template particles) to non-target entities (e.g., monodisperse droplets not containing capture template particles) in the monodisperse emulsion compared to the ratio in the original monodisperse emulsion. Using the method disclosed herein, monodisperse droplets containing capture template particles may be enriched relative to droplets that do not contain one of the capture template particles, e.g., at least 2 fold, at least 3 fold, at least 5 fold, at least 10 fold, at least 20 fold, at least 30 fold, at least 40 fold, at least 50 fold, at least 100 fold, or more.

In some embodiments, excess second fluid is removed from the second mixture including a plurality of capture template particles encapsulated in a plurality of monodisperse droplets after shearing. The excess second fluid can be removed using any suitable method, e.g., by centrifugation and removing the supernatant.

### Fluids Involved in the Generation of Monodisperse Emulsions

As discussed herein, the disclosed methods generally involve combining a plurality of capture template particles with a first fluid to provide a first mixture, wherein the first fluid includes a plurality of target particles; combining the first mixture with a second fluid to provide a third mixture, wherein the second fluid is immiscible with the first fluid; and shearing the third mixture such that a plurality of the capture template particles are encapsulated in a plurality of monodisperse droplets in the second fluid, thereby providing a plurality of monodisperse droplets including the first fluid, reaction buffer, one of the capture template particles, and one of the plurality of target particles. In some embodiments, the methods include the further step of combining a third fluid with the third mixture, following the shearing of the third mixture, to produce a fourth mixture, wherein the third fluid is immiscible with the second fluid.

The first fluid is generally selected to be immiscible with the second fluid and share a common hydrophilicity/hydrophobicity with the material which constitutes the capture template particles. The third fluid is generally selected to be immiscible with the second fluid, and may be miscible or immiscible with the first fluid.

Accordingly, in some embodiments, the first fluid is an aqueous phase fluid; the second fluid is a fluid which is immiscible with the first fluid, such as a non-aqueous phase, e.g., a fluorocarbon, silicone oil, oil, or a hydrocarbon oil, or a combination thereof; and the third fluid is an aqueous phase fluid. Alternatively, in some embodiments the first fluid is a non-aqueous phase, e.g., a fluorocarbon oil, silicone oil, , or a hydrocarbon oil, or a combination thereof; the second fluid is a fluid which is immiscible with the first fluid, e.g., an aqueous phase fluid; and the third fluid is a fluorocarbon oil, silicone oil, or a hydrocarbon oil or a combination thereof.

The non-aqueous phase may serve as a carrier fluid forming a continuous phase that is immiscible with water, or the non-aqueous phase may be a dispersed phase. The non- aqueous phase may be referred to as an oil phase including at least one oil, but may include any liquid (or liquefiable) compound or mixture of liquid compounds that is immiscible with water. The oil may be synthetic or naturally occurring. The oil may or may not include carbon and/or silicon, and may or may not include hydrogen and/or fluorine. The oil may be lipophilic or lipophobic. In other words, the oil may be generally miscible or immiscible with organic solvents. Exemplary oils may include at least one silicone oil, mineral oil, fluorocarbon oil, vegetable oil, or a combination thereof, among others.

In exemplary embodiments, the oil is a fluorinated oil, such as a fluorocarbon oil, which may be a perfluorinated organic solvent. Examples of a suitable fluorocarbon oils include, but are not limited to, C9H5OF15 (HFE-7500), C21F48N2 (FC-40), and perfluoromethyldecalin (PFMD).

As discussed herein, in some embodiments, the first fluid contains a plurality of target particles (e.g. DNA molecules such as genomic DNA molecules, RNA molecules, nucleic acid synthesis reagents such as nucleic acid amplification reagents including PCR and/or isothermal amplification reagents).

In some embodiments, gelling agents may be added to solidify the outer layers of the droplet. Gelling agents include, but are not limited to, gelatin, agar, xanthan gum, gellan gum, carrageenan, isubgol, and guar gum.

### Surfactants

In certain aspects, a surfactant may be included in the first fluid, second fluid, and/or third fluid. Accordingly, a droplet may involve a surfactant stabilized emulsion, e.g., a surfactant stabilized single emulsion or a surfactant stabilized double emulsion, where the surfactant is soluble in the first fluid, second fluid, and/or third fluid. Any convenient surfactant that allows for the desired reactions to be performed in the droplets may be used, including, but not limited to, octylphenol ethoxylate (Triton X-100), polyethylene glycol (PEG), C26H50010 (Tween 20) and/or octylphenoxypolyethoxyethanol (IGEPAL). In other aspects, a droplet is not stabilized by surfactants.

The surfactant used depends on a number of factors such as the oil and aqueous phases (or other suitable immiscible phases, e.g., any suitable hydrophobic and hydrophilic phases) used for the emulsions. For example, when using aqueous droplets in a fluorocarbon oil, the surfactant may have a hydrophilic block (PEG-PPO) and a hydrophobic fluorinated block (Krytox^{®} FSH). If, however, the oil was switched to a hydrocarbon oil, for example, the surfactant may instead be chosen such that it had a hydrophobic hydrocarbon block, like the surfactant ABIL EM90.

Other surfactants can also be envisioned, including ionic surfactants. Other additives can also be included in the oil to stabilize the droplets, including polymers that increase droplet stability at temperatures above 35°C.

Without intending to be bound by any particular theory, it is proposed that the preparation of a thermostable emulsions relies on the use of a surfactant that is able to form membranes or double emulsion interfaces that can withstand high temperatures, such as those associated with standard PCR reactions. One way to accomplish this may be to use a surfactant with a relatively high molecular weight so that when assembled at the interface of a droplet or in a membrane configuration, the energy required to remove the surfactant from the interface (or break the membrane) is higher than can be provided by kT. kT is the amount of heat required to increase the thermodynamic entropy of a system, in natural units, by one nat.

Exemplary surfactants which may be utilized to provide thermostable emulsions are the "biocompatible" surfactants that include PEG-PFPE (polyethyleneglycol- perflouropolyether) block copolymers, e.g., PEG-Krytox^{®} (see, e.g., Holtze et al., "Biocompatible surfactants for water-in-fluorocarbon emulsions," Lab Chip, 2008, 8, 1632-1639), and surfactants that include ionic Krytox^{®} in the oil phase and Jeffamine^{®} (polyetheramine) in the aqueous phase (see, e.g., DeJournette et al., "Creating Biocompatible Oil-Water Interfaces without Synthesis: Direct Interactions between Primary Amines and Carboxylated Perfluorocarbon Surfactants", Anal. Chem. 2013, 85(21):10556-10564). Additional and/or alternative surfactants may be used provided they form stable interfaces. Many suitable surfactants will thus be block copolymer surfactants (like PEG-Krytox^{®}) that have a high molecular weight. These examples include fluorinated molecules and solvents, but it is likely that non-fluorinated molecules can be utilized as well. The term "surfactant" refers to any molecule having both a polar head group, which energetically prefers solvation by water, and a hydrophobic tail that is not well solvated by water. The presently disclosed methods are not limited to a particular surfactant. A variety of surfactants are contemplated including, but not limited to, nonionic and ionic surfactants (e.g., TRITON X-100; TWEEN 20; and TYLOXAPOL) or combinations thereof.

Accordingly, in some embodiments, the present disclosure provides thermostable emulsions. These emulsions are suitable for use in performing biological reactions, such as PCR, RT-PCR, protein-protein interaction studies, etc.

### Shearing

To generate a monodisperse emulsion, the disclosed methods include a step of shearing the second mixture provided by combining the first mixture with a third fluid immiscible with the first fluid. Any suitable method or technique may be utilized to apply a sufficient shear force to the second mixture. For example, the second mixture may be sheared by flowing the second mixture through a pipette tip. Other methods include, but are not limited to, shaking the second mixture with a homogenizer (e.g., vortexer), or shaking the second mixture with a bead beater. The application of a sufficient shear force breaks the second mixture into monodisperse droplets that encapsulate one of a plurality of capture template particles. There may also be some droplets that do not contain one of the plurality of capture template particles.

Generally, if the shear is increased, the average droplet size generated will be lower than that of the size of the capture template particles. However, since the capture template particles are in solid form, the droplets containing them will not be any smaller in size, thereby generating a monodisperse emulsion. If the shear rate is substantially higher than the modulus of the capture template particle, then the shear can squeeze liquid out of the capture template particles. Without intending to be bound by any particular theory, it is proposed that a suitable shear rate is one which matches appropriately the modulus of the capture template particles. For example, it may be desirable to select a shear rate/force higher than the Laplace pressure of the droplets of the desired size but less than the modulus of the template particles

### Adding Reagents to Single-Emulsion Droplets, Multiple-Emulsion Droplets and/or GUVs

In practicing the subject methods, a number of reagents may need to be added to the droplets, in one or more steps (e.g., about 2, about 3, about 4, or about 5 or more steps). The means of adding reagents to the droplets may vary in a number of ways depending for example, on the emulsification stage of the droplets, e.g., different approaches may be applicable to the addition of reagents to monodisperse single-emulsion droplets relative to multiple-emulsion droplets, such as double emulsion droplets. Approaches of interest include, but are not limited to, those described by Ahn, et al., Appl. Phys. Lett. 88, 264105 (2006); Priest, et al., Appl. Phys. Lett. 89, 134101 (2006); Abate, et al., PNAS, November 9, 2010 vol. 107 no. 45 19163-19166; and Song, et al., Anal.Chem., 2006, 78 (14), pp 4839-4849. In some embodiments, reagents may be added to droplets during the emulsification process as described herein, e.g., as components of the first fluid, e.g., without the use of a microfluidic device or system. In other embodiments, microfluidic techniques, devices and/or systems may be utilized to add reagents and/or modify monodisperse droplets once prepared as otherwise described herein.

For instance, a reagent may be added to a monodisperse single-emulsion droplet as described herein by a method involving merging a droplet with a second droplet that contains the reagent(s). The reagent(s) that are contained in the second droplet may be added by any convenient means, specifically including those described herein. This droplet may be merged with the first droplet to create a droplet that includes the contents of both the first droplet and the second droplet. In some embodiments, the first droplet is substantially larger than the second droplet and outnumbers the second droplet.

One or more reagents may also, or instead, be added to monodisperse single-emulsion droplets as described herein using techniques such as droplet coalescence, and/or picoinjection. In droplet coalescence, a target droplet may be flowed alongside a droplet containing the reagent(s) to be added to the target droplet. The two droplets may be flowed such that they are in contact with each other, but not touching other droplets.

These droplets may then be passed through electrodes or other means of applying an electrical field, wherein the electric field may destabilize the droplets such that they are merged together.

In picoinjection, a target droplet may be flowed past a channel containing the reagent(s) to be added, wherein the reagent(s) are at an elevated pressure. Due to the presence of the surfactants, however, in the absence of an electric field, the droplet will flow past without being injected, because surfactants coating the droplet may prevent the fluid(s) from entering. However, if an electric field is applied to the droplet as it passes the injector, fluid containing the reagent(s) will be injected into the droplet. The amount of reagent added to the droplet may be controlled by several different parameters, such as by adjusting the injection pressure and the velocity of the flowing drops, by switching the electric field on and off, and the like.

In other aspects, one or more reagents may also, or instead, be added to a monodisperse single-emulsion droplet as described herein by a method that does not rely on merging two droplets together or on injecting liquid into a droplet. Rather, one or more reagents may be added to a droplet by a method involving the steps of emulsifying a reagent into a stream of very small drops, and merging these small drops with a target droplet. Such methods are referred to herein as "reagent addition through multiple-drop coalescence." These methods take advantage of the fact that due to the small size of the drops to be added compared to that of the target droplet, the small drops will flow faster than the target droplets and collect behind them. The collection can then be merged by, for example, applying an electric field. This approach can also, or instead, be used to add multiple reagents to a droplet by using several co-flowing streams of small drops of different fluids. To enable effective merger of the tiny and target droplets, it is important to make the tiny drops smaller than the channel containing the target droplets, and also to make the distance between the channel injecting the target droplets from the electrodes applying the electric field sufficiently long so as to give the tiny drops time to "catch up" to the target droplets. If this channel is too short, not all tiny drops will merge with the target droplet and less than the desired amount of reagent may be added. To a certain degree, this can be compensated for by increasing the magnitude of the electric field, which tends to allow drops that are farther apart to merge. In addition to making the tiny drops on the same microfluidic device, they can also, or instead, be made offline using another microfluidic drop maker or through homogenization and then injecting them into the device containing the target droplets.

Accordingly, in certain aspects a reagent is added to a droplet prepared as described herein by a method involving emulsifying the reagent into a stream of droplets, wherein the droplets are smaller than the size of the target droplets (e.g., monodisperse single- emulsion droplets or multiple-emulsion droplets or GUVs); flowing the droplets together with the target droplets; and merging a droplet with the target droplet. The diameter of the droplets contained in the stream of droplets may vary ranging from about 75% or less than that of the diameter of the target droplet, e.g., the diameter of the flowing droplets is about 75% or less than that of the diameter of the target droplet, about 50% or less than that of the diameter of the target droplet, about 25% or less than that of the diameter of the target droplet, about 15% or less than that of the diameter of the target droplet, about 10% or less than that of the diameter of the target droplet, about 5% or less than that of the diameter of the target droplet, or about 2% or less than that of the diameter of the target droplet. In certain aspects, a plurality of flowing droplets may be merged with the target droplet, such as 2 or more droplets, 3 or more, 4 or more, or 5 or more. Such merging may be achieved by any convenient means, including but not limited to by applying an electric field, wherein the electric field is effective to merge the flowing droplet with the target droplet.

As a variation of the above-described methods, the fluids may be jetting. That is, rather than emulsifying the fluid to be added into flowing droplets, a long jet of this fluid can be formed and flowed alongside the target droplet. These two fluids can then be merged by, for example, applying an electric field. The result is a jet with bulges where the droplets are, which may naturally break apart into droplets of roughly the size of the target droplets before the merger, due to the Rayleigh plateau instability. A number of variants are contemplated. For instance, one or more agents may be added to the jetting fluid to make it easier to jet, such as gelling agents and/or surfactants. Moreover, the viscosity of the continuous fluid could also be adjusted to enable jetting, such as that described by Utada, et al., Phys. Rev. Lett. 99, 094502 (2007).

In other aspects, one or more reagents may be added using a method that uses the injection fluid itself as an electrode, by exploiting dissolved electrolytes in solution.

In another aspect, a reagent is added to a droplet formed at an earlier time by enveloping the droplet to which the reagent is to be added (i.e., the "target droplet") inside a drop containing the reagent to be added (the "target reagent"). In certain embodiments such a method is carried out by first encapsulating the target droplet in a shell of a suitable hydrophobic phase, e.g., oil, to form a double emulsion. The double emulsion is then encapsulated by a droplet containing the target reagent to form a triple emulsion. To combine the target drop with the drop containing the target reagent, the double emulsion is then burst open using any suitable method, including, but not limited to, applying an electric field, adding chemicals that destabilizes the droplet interface, flowing the triple emulsion through constrictions and other microfluidic geometries, applying shearing or ultrasound, increasing or reducing temperature, or by encapsulating magnetic particles in the droplet that can rupture the double emulsion interface when pulled by a magnetic field.

Aspects of the above-described methods of adding reagents to droplets are described in more detail in U.S. Patent Application Publication No. 2015/0232942.

### Lysis

In some embodiments, one or more lysing agents may also be added to the monodisperse single-emulsion droplets, multiple-emulsion droplets, and/or GUVs containing a cell, under conditions in which the cell(s) may be caused to burst, thereby releasing their cellular contents, such as genomes. The lysing agents may be added after the cells are encapsulated into monodisperse single-emulsion droplets, multiple-emulsion droplets, and/or GUVs. Any convenient lysing agent may be employed, such as proteinase K or cytotoxins. In particular embodiments, cells may be co-encapsulated in monodisperse single-emulsion droplets, multiple-emulsion droplets, and/or GUVs with lysis buffer containing detergents such as Triton X-100 and/or proteinase K. The specific conditions in which the cell(s) may be caused to burst will vary depending on the specific lysing agent used. For example, if proteinase K is incorporated as a lysing agent, the monodisperse single-emulsion droplets, multiple- emulsion droplets, and/or GUVs may be heated to about 37-60°C for about 20 min to lyse the cells and to allow the proteinase K to digest cellular proteins, after which they may be heated to about 95°C for about 5-10 min to deactivate the proteinase K.

In some embodiments, the lysis agent/s, (unlimiting examples of a lysis agent include Triton X-100, SDS, or Sarkosyl) is present in the second fluid and triggers cell lysis upon emulsification (shearing of the third mixture). Co-dissolution of the lysis agent and the second fluid (e.g. fluorocarbon oil, silicone oil, or a hydrocarbon oil, or a combination thereof) may be facilitated by a micellization process, optionally with the addition of any suitable surfactant to encapsulate the lysis agent. Unlimiting examples of surfactants are described in the present disclosure. Alternatively, the co-dissolution of the lysis agent and the second fluid may be facilitated with the use of synthetically modified micro/nano capsules such as paraffin wax micro/nano particles (e.g. capsules), thereby generating paraffin wax nanoparticles and/or microparticles which contain the lysis agent/s.

In some embodiments, the lysis process may also be triggered directly by suitable surfactants contained (for example, in capsule form) in the second fluid, such as without limitation, perfluoropolyether (PFPE) with ionic end group, carboxylic acid, ammonium carboxylate, potassium carboxylate, or combinations thereof.

In certain aspects, cell lysis may also, or instead, rely on techniques that do not involve addition of lysing agent. For example, lysis may be achieved by mechanical techniques that may employ various geometric features to effect piercing, shearing, abrading, etc. of cells. Other types of mechanical breakage such as acoustic techniques may also be used. Further, thermal energy can also be used to lyse cells. Any convenient means of effecting cell lysis may be employed in the methods described herein.

### PCR

As summarized above, in practicing methods of the present disclosure a PCR-based assay may be used to detect the presence of certain nucleic acids of interest, e.g., genes of interest and/or genetic markers, e.g., oncogene(s), present in cells or a heterogeneous sample of nucleic acids. Such PCR based assays may be performed in the same monodisperse droplet, e.g., monodisperse single-emulsion droplet or multiple emulsion monodisperse droplet as a previous or subsequent MDA amplification step. In other embodiments, PCR reactions may be conducted in monodisperse droplets independently. The conditions of such PCR-based assays may vary in one or more ways.

For instance, the number of PCR primers that may be added to a monodisperse single-emulsion droplet or multiple-emulsion droplet and/or GUV may vary. The term "primer" may refer to more than one primer and refers to an oligonucleotide, whether occurring naturally, as in a purified restriction digest, or produced synthetically, which is capable of acting as a point of initiation of synthesis along a complementary strand when placed under conditions in which synthesis of a primer extension product which is complementary to a nucleic acid strand is catalyzed. Such conditions include the presence of four different deoxyribonucleoside triphosphates and a polymerization- inducing agent such as DNA polymerase or reverse transcriptase, in a suitable buffer ("buffer" includes substituents which are cofactors, or which affect pH, ionic strength, etc.), and at a suitable temperature. The primer is preferably singlestranded for maximum efficiency in amplification.

The complement of a nucleic acid sequence as used herein refers to an oligonucleotide which, when aligned with the nucleic acid sequence such that the 5' end of one sequence is paired with the 3' end of the other, is in "antiparallel association." Complementarity need not be perfect; stable duplexes may contain mismatched base pairs or unmatched bases. Those skilled in the art of nucleic acid technology can determine duplex stability empirically considering a number of variables including, for example, the length of the oligonucleotide, percent concentration of cytosine and guanine bases in the oligonucleotide, ionic strength, and incidence of mismatched base pairs.

Primers that may be added to a monodisperse single-emulsion droplet or multiple-emulsion droplet and/or GUV may range from about 1 to about 500 or more, e.g., about 2 to 100 primers, about 2 to 10 primers, about 10 to 20 primers, about 20 to 30 primers, about 30 to 40 primers, about 40 to 50 primers, about 50 to 60 primers, about 60 to 70 primers, about 70 to 80 primers, about 80 to 90 primers, about 90 to 100 primers, about 100 to 150 primers, about 150 to 200 primers, about 200 to 250 primers, about 250 to 300 primers, about 300 to 350 primers, about 350 to 400 primers, about 400 to 450 primers, about 450 to 500 primers, or about 500 primers or more.

These primers may contain primers for one or more gene of interest, e.g. oncogenes. The number of primers for genes of interest that are added may be from about one to 500, e.g., about 1 to 10 primers, about 10 to 20 primers, about 20 to 30 primers, about 30 to 40 primers, about 40 to 50 primers, about 50 to 60 primers, about 60 to 70 primers, about 70 to 80 primers, about 80 to 90 primers, about 90 to 100 primers, about 100 to 150 primers, about 150 to 200 primers, about 200 to 250 primers, about 250 to 300 primers, about 300 to 350 primers, about 350 to 400 primers, about 400 to 450 primers, about 450 to 500 primers, or about 500 primers or more. Genes and oncogenes of interest include, but are not limited to, BAX, BCL2L1, CASP8, CDK4, ELK1, ETS1, HGF, JAK2, JUNB, JUND, KIT, KITLG, MCL1, MET, MOS, MYB, NFKBIA, EGFR, Myc, EpCAM, NRAS, PIK3CA, PML, PRKCA, RAF1, RARA, REL, ROS1, RUNX1, SRC, STAT3, CD45, cytokeratins, CEA, CD133, HER2, CD44, CD49f, CD146, MUC1/2, ABL1, AKT1, APC, ATM, BRAF, CDH1, CDKN2A, CTNNB1, EGFR, ERBB2, ERBB4, EZH2, FBXW7, FGFR2, FGFR3, FLT3, GNAS, GNAQ, GNA11, HNF1A, HRAS, IDH1, IDH2, JAK2, JAK3, KDR, KIT, KRAS, MET, MLH1, NOTCH1, NPM1, NRAS, PDGFRA, PIK3CA, PTEN, PTPN11, RB1, RET, SMAD4, STK11, TP53, VHL, and ZHX2.

Such primers and/or reagents may be added to a monodisperse single-emulsion droplet or multiple-emulsion droplet and/or GUV in one step, or in more than one step. For instance, the primers may be added in two or more steps, three or more steps, four or more steps, or five or more steps. Regardless of whether the primers are added in one step or in more than one step, they may be added after the addition of a lysing agent, prior to the addition of a lysing agent, or concomitantly with the addition of a lysing agent. When added before or after the addition of a lysing agent, the PCR primers may be added in a separate step from the addition of a lysing agent.

Once primers have been added to a monodisperse single-emulsion droplet or multiple-emulsion droplet and/or GUV the monodisperse single-emulsion droplet or multiple- emulsion monodisperse droplet and/or GUV may be incubated under conditions allowing for PCR. In some embodiments, the monodisperse single-emulsion droplet or multiple-emulsion droplet and/or GUV may be incubated on the same microfluidic device as was used to add the primer(s), or may be incubated on a separate device. In certain embodiments, incubating the monodisperse single-emulsion droplet or multiple-emulsion droplet and/or GUV under conditions allowing for PCR amplification is performed on the same microfluidic device used to encapsulate and lyse cells. In some embodiments, any one of the techniques/steps described above, including multiple-emulsion droplet and/or GUV incubation, addition of the primer(s), and PCR amplification, is done without requiring the use of a microfluidic device/s. Incubating the monodisperse single-emulsion droplet or multiple-emulsion droplet and/or GUV may take a variety of forms. In certain aspects, the monodisperse single-emulsion droplet or multiple-emulsion droplet and/or GUV containing the PCR mix may be flowed through a channel that incubates the monodisperse droplets under conditions effective for PCR. In some embodiments, PCR reactions are performed without the use of microfluidic devices and/or systems. In some embodiments, flowing the monodisperse single-emulsion droplet or multiple-emulsion droplet and/or GUV through a channel may involve a channel that snakes over various temperature zones maintained at temperatures effective for PCR. Such channels may, for example, cycle over two or more temperature zones, wherein at least one zone is maintained at about 65°C and at least one zone is maintained at about 95°C. Alternatively, zones for 86°C, 60°C and 20°C may be utilized. As the monodisperse single-emulsion droplets or multiple-emulsion and/or GUVs move through such zones, their temperature cycles, as needed for PCR. The precise number of zones, and the respective temperature of each zone, may be readily determined by those of skill in the art to achieve the desired PCR amplification.

### Suitable Subjects and/or Samples

The described methods may be applied to biological samples taken from a variety of different subjects. In many embodiments the subjects are "mammals" or "mammalian", where these terms are used broadly to describe organisms which are within the class mammalia, including the orders carnivore (e.g., dogs and cats), rodentia (e.g., mice, guinea pigs, and rats), and primates (e.g., humans, chimpanzees, and monkeys). In many embodiments, the subjects are humans. The subject methods may be applied to human subjects of both genders and at any stage of development (i.e., neonates, infant, juvenile, adolescent, adult), where in certain embodiments the human subject is a juvenile, adolescent or adult. While the present disclosure may be applied to a human subject, it is to be understood that the subject methods may also be carried-out on other animal subjects (that is, in "non-human subjects") such as, but not limited to, birds, mice, rats, dogs, cats, livestock and horses. Accordingly, it is to be understood that any subject in need of assessment as described herein is suitable.

Moreover, suitable subjects include those who have and those who have not been diagnosed with a condition, such as cancer. Suitable subjects include those that are and are not displaying clinical presentations of one or more cancers. In certain aspects, a subject may one that may be at risk of developing cancer, due to one or more factors such as family history, chemical and/or environmental exposure, genetic mutation(s) (e.g., BRCA1 and/or BRCA2 mutation), hormones, infectious agents, radiation exposure, lifestyle (e.g., diet and/or smoking), presence of one or more other disease conditions, and the like.

As described more fully above, a variety of different types of biological samples may be obtained from such subjects. In certain embodiments, whole blood is extracted from a subject. When desired, whole blood may be treated prior to practicing the subject methods, such as by centrifugation, fractionation, purification, and the like. The volume of the whole blood sample that is extracted from a subject may be 100 mL or less, e.g., about 100 mL or less, about 50 mL or less, about 30 mL or less, about 15 mL or less, about 10 mL or less, about 5 mL or less, or about 1 mL or less.

The subject methods and devices as described herein are compatible with both fixed and live cells. In certain embodiments, the subject methods and devices are practiced with live cells. In other embodiments, the subject methods and devices are practiced with fixed cells. Fixing a cellular sample allows for the sample to be washed to extract small molecules and lipids that may interfere with downstream analysis. Further, fixing and permeabilizing cells allows the cells to be stained with antibodies for surface proteins as well as intracellular proteins. Combined with the nucleic amplification methods as described herein, such staining can be used to achieve high levels of multiplexing because the antibodies are localized to the cell sample, while the nucleic amplification products are free within a monodisperse single-emulsion droplet, multiple-emulsion monodisperse droplet, and/or GUV. Such a configuration allows for dyes of the same color to be used for antibodies and for amplicons produced by nucleic acid amplification. Any suitable method can be used to fix cells including, but not limited to, fixation by using aqueous solutions such as formaldehyde, methanol and/or acetone. Fixed cells may be further embedded in solid support blocks, such as those composed of paraffin, enabling long-term storage. In some embodiments, in order to perform the subject methods on cells which have been fixed and embedded as described above, said cells are deparaffinized, sectioned, and permeabilized. Deparaffinization may be done using any method known in the art including, without limitation, washes with xylene and/or ethanol. Permeabilization may be done using any method known in the art including, without limitation, washes with such reagents as Acetone, Methanol, Triton-X, Tween 20, and NP-40.

### Examples of Non-Limiting Aspects of the Disclosure

Aspects, including embodiments, of the present subject matter described above may be beneficial alone or in combination, with one or more other aspects or embodiments.

## Claims

1. A method of target capture in monodisperse droplets comprising:
combining a plurality of capture template particles comprising a hydrogel and further comprising one or more tethering moieties, wherein each of the tethering moieties are attachable to one or more target particles, with a first fluid to provide a first mixture, wherein the first fluid comprises a plurality of target particles;
incubating the first mixture to allow association of the plurality of target particles to the plurality of capture template particles, thereby a portion of the plurality of target particles become associated to the capture template particles;
generating a supernatant and pellet from the first mixture, optionally by centrifuging;
removing the supernatant to separate the plurality of capture template particles and associated target particles from the solution of the first mixture;
washing and resuspending the pellet in a reaction buffer to provide a second mixture;
combining the second mixture with a second fluid to provide a third mixture, wherein the second fluid is immiscible with the second mixture; and
shearing the third mixture such that a plurality of the capture template particles are encapsulated in a plurality of monodisperse droplets in the second fluid, thereby providing a plurality of monodisperse droplets comprising reaction buffer, one of the capture template particles, and one of the plurality of target particles associated to said capture template particles.

2. The method of claim 1, wherein the second fluid comprises an oil.

3. The method of claim 2, wherein the oil comprises a fluorocarbon oil, a silicone oil, a hydrocarbon oil, or a combination thereof.

4. The method of any one of claims 1 to 3, wherein the first fluid comprises a biological sample.

5. The method of claim 4, wherein the biological sample is a body fluid.

6. The method of claim 5, wherein the body fluid comprises target particles selected from the group consisting of circulating cells, cellular components, cell-free nucleic acids, extracellular vesicles, protein antigens, prokaryotic cells, fungi, viruses, and combinations thereof.

7. The method of claim 6, wherein the body fluid is a human body fluid.

8. The method of claim 1, wherein the association of the plurality of target particles to the plurality of capture template particles is target-specific.

## Patentansprüche

1. Verfahren zum Einfangen von Zielen in monodispersen Tröpfchen, umfassend:
Kombinieren einer Vielzahl von Einfangtemplatpartikeln, die ein Hydrogel und ferner eine oder mehrere Bindungseinheiten umfassen, wobei jede der Bindungseinheiten an ein oder mehrere Zielpartikel gebunden werden kann, mit einem ersten Fluid, um eine erste Mischung bereitzustellen, wobei das erste Fluid eine Vielzahl von Zielpartikeln umfasst;
Inkubieren der ersten Mischung, um eine Assoziation der Vielzahl von Zielpartikeln mit der Vielzahl von Einfangtemplatpartikeln zu ermöglichen, wodurch ein Teil der Vielzahl von Zielpartikeln mit den Einfangtemplatpartikeln assoziiert wird;
Erzeugen eines Überstands und eines Pellets aus der ersten Mischung, optional durch Zentrifugieren;
Entfernen des Überstands, um die Vielzahl von Einfangtemplatpartikeln und assoziierten Zielpartikeln von der Lösung der ersten Mischung zu trennen;
Waschen und Resuspendieren des Pellets in einem Reaktionspuffer, um eine zweite Mischung bereitzustellen;
Kombinieren der zweiten Mischung mit einem zweiten Fluid, um eine dritte Mischung bereitzustellen, wobei das zweite Fluid mit der zweiten Mischung nicht mischbar ist; und
Scheren der dritten Mischung, so dass eine Vielzahl der Einfangtemplatpartikel in einer Vielzahl von monodispersen Tröpfchen in dem zweiten Fluid eingekapselt wird, wodurch eine Vielzahl von monodispersen Tröpfchen bereitgestellt wird, die Reaktionspuffer, eines der Einfangtemplatpartikel und eines der Vielzahl von Zielpartikeln, die mit den Einfangtemplatpartikeln assoziiert sind, umfassen.

2. Verfahren nach Anspruch 1, wobei das zweite Fluid ein Öl umfasst.

3. Verfahren nach Anspruch 2, wobei das Öl ein Fluorkohlenstofföl, ein Silikonöl, ein Kohlenwasserstofföl oder eine Kombination davon umfasst.

4. Verfahren nach einem der Ansprüche 1 bis 3, wobei das erste Fluid eine biologische Probe umfasst.

5. Verfahren nach Anspruch 4, wobei die biologische Probe eine Körperflüssigkeit ist.

6. Verfahren nach Anspruch 5, wobei die Körperflüssigkeit Zielpartikel umfasst, die aus der Gruppe ausgewählt sind, die aus zirkulierenden Zellen, zellulären Komponenten, zellfreien Nukleinsäuren, extrazellulären Vesikeln, Proteinantigenen, prokaryotischen Zellen, Pilzen, Viren und Kombinationen davon besteht.

7. Verfahren nach Anspruch 6, wobei die Körperflüssigkeit eine menschliche Körperflüssigkeit ist.

8. Verfahren nach Anspruch 1, wobei die Assoziation der Vielzahl von Zielpartikeln zu der Vielzahl von Einfangtemplatpartikeln zielspezifisch ist.

## Revendications

1. Procédé de capture de cible dans des gouttelettes monodispersées comprenant :
la combinaison d'une pluralité de particules modèles de capture comprenant un hydrogel et comprenant en outre une ou plusieurs fragments de fixation, dans lequel chacun des fragments de fixation peut être attaché à une ou plusieurs particules cibles, avec un premier fluide pour obtenir un premier mélange, dans lequel le premier fluide comprend une pluralité de particules cibles ;
l'incubation du premier mélange pour permettre une association de la pluralité de particules cibles à la pluralité de particules modèles de capture, de sorte qu'une partie de la pluralité de particules cibles soit associée aux particules modèles de capture ;
la production d'un surnageant et d'un culot à partir du premier mélange, éventuellement par centrifugation ;
l'élimination du surnageant pour séparer la pluralité de particules modèles de capture et de particules cibles associées de la solution du premier mélange ;
le lavage et la remise en suspension du culot dans un tampon de réaction pour obtenir un deuxième mélange ;
la combinaison du deuxième mélange avec un second fluide pour obtenir un troisième mélange, dans lequel le second fluide est immiscible avec le deuxième mélange ; et
le cisaillement du troisième mélange de manière à ce qu'une pluralité de particules modèles de capture soient encapsulées dans une pluralité de gouttelettes monodispersées dans le second fluide, fournissant ainsi une pluralité de gouttelettes monodispersées comprenant un tampon de réaction, une des particules modèles de capture, et une de la pluralité de particules cibles associées auxdites particules modèles de capture.

2. Procédé selon la revendication 1, dans lequel le second fluide est une huile.

3. Procédé selon la revendication 2, dans lequel l'huile comprend une huile fluorocarbonée, une huile de silicone, une huile hydrocarbure ou une combinaison de celles-ci.

4. Procédé selon l'une quelconque des revendications 1 à 3, dans lequel le premier fluide comprend un échantillon biologique.

5. Procédé selon la revendication 4, dans lequel l'échantillon biologique est un fluide corporel.

6. Procédé selon la revendication 5, dans lequel le fluide corporel comprend des particules cibles choisies dans le groupe constitué de cellules circulantes, composants cellulaires, acides nucléiques acellulaires, vésicules extracellulaires, antigènes protéiques, cellules procaryotes, champignons, virus, et combinaisons de ceux-ci.

7. Procédé selon la revendication 6, dans lequel le fluide corporel est un fluide corporel humain.

8. Procédé selon la revendication 1, dans lequel l'association de la pluralité de particules cibles à la pluralité de particules modèles de capture est spécifique à la cible.
